(19)

Europäisches Patentamt

European Patent Office

Office européen des brevets

(11)  **EP 4 014 994 A1**

(12)  **EUROPEAN PATENT APPLICATION**
published in accordance with Art. 153(4) EPC

(43) Date of publication:
**22.06.2022  Bulletin 2022/25**

(21) Application number: **20852963.6**

(22) Date of filing: **06.08.2020**

(51) International Patent Classification (IPC):
**A61K 39/395** (2006.01)     **A61K 45/00** (2006.01)
**A61P 19/08** (2006.01)       **A61P 29/00** (2006.01)
**A61P 35/00** (2006.01)       **A61P 35/04** (2006.01)
**A61P 37/06** (2006.01)       **A61P 37/08** (2006.01)
**A61P 43/00** (2006.01)       **C07K 14/78** (2006.01)
**C07K 16/00** (2006.01)       **C07K 16/28** (2006.01)
**C07K 16/30** (2006.01)       **C07K 19/00** (2006.01)
**C12M 1/34** (2006.01)        **C12N 15/12** (2006.01)
**C12Q 1/04** (2006.01)        **A61K 38/10** (2006.01)
**G01N 33/53** (2006.01)

(52) Cooperative Patent Classification (CPC):
**A61K 38/10; A61K 39/395; A61K 45/00;**
**A61P 19/08; A61P 29/00; A61P 35/00;**
**A61P 35/04; A61P 37/06; A61P 37/08;**
**A61P 43/00; C07K 14/78; C07K 16/00;**
**C07K 16/28; C07K 16/30; C07K 19/00;**     (Cont.)

(86) International application number:
**PCT/JP2020/030175**

(87) International publication number:
**WO 2021/029318 (18.02.2021 Gazette 2021/07)**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority:  **13.08.2019  JP 2019148423**

(71) Applicant: **Tohoku University**
**Sendai-shi, Miyagi 980-8577 (JP)**

(72) Inventors:
• **TAKAI Toshiyuki**
  **Sendai-shi, Miyagi 980-8577 (JP)**
• **INUI Masanori**
  **Sendai-shi, Miyagi 980-8577 (JP)**
• **SU Mei tzu**
  **Sendai-shi, Miyagi 980-8577 (JP)**
• **ENDO Shota**
  **Sendai-shi, Miyagi 980-8577 (JP)**

(74) Representative: **Cabinet Becker et Associés**
**25, rue Louis le Grand**
**75002 Paris (FR)**

(54)  **IMMUNE CHECKPOINT INHIBITOR, THERAPEUTIC AGENT FOR IMMUNE CHECKPOINT-RELATED DISEASE, IMMUNOSUPPRESSANT, ANTI-FIBRONECTIN ANTIBODY OR DERIVATIVE THEREOF, FIBRONECTIN ANALOG, KIT FOR DETECTING FIBRONECTIN OR PARTIAL PROTEIN THEREOF, AND METHOD FOR DETECTING FIBRONECTIN OR PARTIAL PROTEIN THEREOF**

(57)    An immune checkpoint inhibitor containing a substance that inhibits binding between fibronectin and an immunosuppressive receptor LILRB4 as an active ingredient, a therapeutic agent for an immune checkpoint-related disease, an immunosuppressant containing a substance that activates an immunosuppressive receptor LILRB4 as an active ingredient, an anti-fibronectin antibody or a derivative thereof, a fibronectin analog, a kit for detecting fibronectin or a partial protein thereof, and a method for detecting fibronectin or a partial protein thereof in a biological sample using the kit.

EP 4 014 994 A1

[FIG. 15]

(52) Cooperative Patent Classification (CPC): (Cont.)
**C12M 1/34; C12Q 1/04; G01N 33/53**

SEQUENCE LISTING

<110> Tohoku university

<120> Immune checkpoint inhibitor, therapeutic agent for immune checkpoint-related disease, immunosuppressant, anti-fibronectin antibody or derivative thereof, fibronectin analog, kit for detecting fibronectin or partial protein thereof and method for detecting fibronectin or partial protein thereof

<130> PC-30385

<140> JP 2019-148423
<141> 2019-08-13

<160> 3

<170> PatentIn version 3.5

<210> 1
<211> 20
<212> PRT
<213> Homo sapiens

<400> 1

Cys Thr Cys Ile Gly Ala Gly Arg Gly Arg Ile Ser Cys Thr Ile Ala
1               5                   10                  15


Asn Arg Cys His
            20


<210> 2
<211> 31
<212> DNA
<213> Artificial Sequence

<220>
<223> FN30 forward primer

<400> 2
atagaattcg cagtccccgg tggctgtcag t                          31


<210> 3
<211> 30
<212> DNA
<213> Artificial Sequence

<220>
<223> FN30 reverse primer

<400> 3
ttaagatctt ccgctcgatg tggtctgcac                            30


**Description**

TECHNICAL FIELD

The present invention relates to an immune checkpoint inhibitor, a therapeutic agent for an immune checkpoint-related disease, an immunosuppressant, an anti-fibronectin antibody or a derivative thereof, a fibronectin analog, a kit for detecting fibronectin or a partial protein thereof, and a method for detecting fibronectin or a partial protein thereof.
The present application claims priority based on Japanese Patent Application No. 2019-148423 filed in Japan on August 13, 2019, the contents of which are incorporated herein by reference.

BACKGROUND ART

In recent years, cancer immunotherapy using an immune checkpoint inhibitor has attracted attention. The immune checkpoint inhibitor is an agent that cancels the suppression of activation of T cells by an immune checkpoint molecule having an action of suppressing the immune response to itself and suppressing an excessive immune reaction by binding to the immune checkpoint molecule or a ligand thereof so as to inhibit immunosuppressive signal transduction.
The inventors of the present application found that by using LILRB4 (leukocyte Ig-like receptor B4, hereinafter also referred to as B4) that is an immunosuppressive receptor for an unknown ligand, an inflammatory disease derived from infection or autoimmunity can be determined (PTL 1).
As the ligand for LILRB4, recently, CD166, ApoE, Angptls, and the like have been reported (NPLs 1 to 3). However, it cannot be said that the proof that these are physiological ligands is sufficient.
Fibronectin (hereinafter also referred to as FN) is a glycoprotein of about 259 kDa present in an extracellular matrix (hereinafter also referred to as ECM), on a cell surface, and in a body fluid. Fibronectin is divided into 6 regions (domains) by a treatment with thermolysin that is a protease. These are called 1. a fibrin/heparin binding region (fibrin/heparin binding-FN), 2. a collagen binding region (collagen binding-FN), 3. a heparin binding region (heparin binding-FN), 4. a cell/integrin binding region (cell/integrin-binding-domain (CBD)-FN), 5. a second heparin binding region (second heparin binding-FN), and 6. a second fibrin binding region (second fibrin binding-FN) based on their specific molecular binding capacities. In this manner, FN is constituted by multiple domains having different binding capacities to physiological molecules. It has been reported so far that in some autoimmune diseases such as systemic lupus erythematosus (SLE) and rheumatoid arthritis (RA), variations in FN concentration in plasma or a body fluid (for example, joint fluid) are observed, and also that a domain analysis for evaluating FN fragmentation using a monoclonal antibody is useful for disease diagnosis or severity evaluation (NPLs 4 to 6). In particular, the concentration of fibrin/heparin binding-FN is $24 \pm 12$ $\mu$g/mL (p<0.003) in SLE and $36 \pm 22$ $\mu$g/mL (p<0.00002) in RA in comparison to that of healthy subjects ($61 \pm 18$ $\mu$g/mL), and is expected to be used for diagnosis. Further, it has been reported that FN promotes the metastatic potential and invasive potential of a lung cancer cell line (NPL 7). However, none of the above literature has reported that FN is a ligand for LILRB4.

Citation List

Patent Literature

PTL 1: JP-A-2018-25554

Non Patent Literature

NPL 1: J Immunol. 2018 Feb 1; 200(3): 1207-1219
NPL 2: Blood. 2014 Aug 7; 124(6): 924-935
NPL 3: Nature. 2018 Oct; 562(7728): 605-609
NPL 4: Rheumatology (Oxford). 2007 Jul; 46(7): 1071-1075
NPL 5: J Rheumatol. 1987 Oct; 14(5): 1052-1054
NPL 6: Rheumatol Int. 2013 Jan; 33(1): 37-43
NPL 7: Br J Cancer 2009 Jul 21; 101(2): 327-334

SUMMARY OF INVENTION

Technical Problem

An object of the present invention is to provide an immune checkpoint inhibitor, a therapeutic agent for an immune checkpoint-related disease, an immunosuppressant, an anti-fibronectin antibody or a derivative thereof, a fibronectin analog, a kit for detecting fibronectin or a partial protein thereof, and a method for detecting fibronectin or a partial protein thereof.

Solution to Problem

The present inventors found that a physiological ligand for LILRB4 is fibronectin that is one of the main constituent proteins of ECM, identified a target sequence of LILRB4 in the fibronectin, and found that a substance that inhibits binding between the target sequence and the fibronectin is useful as an immune checkpoint inhibitor, a therapeutic agent for an immune checkpoint-related disease, and an immunosuppressant, and thus completed the present invention.
The present invention includes the following aspects.

[1] An immune checkpoint inhibitor containing a substance that inhibits binding between fibronectin and an immunosuppressive receptor LILRB4 as an active ingredient.
[2] The immune checkpoint inhibitor according to [1], wherein the fibronectin binds to the immunosuppressive receptor LILRB4 via the amino acid sequence represented by SEQ ID NO: 1 in the fibronectin.
[3] The immune checkpoint inhibitor according to [1] or [2], wherein the substance that inhibits binding between the fibronectin and the immunosuppressive receptor LILRB4 is an anti-fibronectin antibody or a derivative thereof, an anti-immunosuppressive receptor LILRB4 antibody or a derivative thereof, or a fibronectin analog.
[4] The immune checkpoint inhibitor according to [3], wherein the anti-fibronectin antibody or a derivative thereof binds to the amino acid sequence represented by SEQ ID NO: 1 in the fibronectin.
[5] The immune checkpoint inhibitor according to [3], wherein the fibronectin analog is any one of the following peptides (a) to (c):

(a) a peptide containing the amino acid sequence represented by SEQ ID NO: 1;
(b) a peptide containing an amino acid sequence in which one to several amino acids have been deleted, inserted, substituted, or added in the amino acid sequence represented by SEQ ID NO: 1, and having a binding capacity to a fibronectin binding site of the immunosuppressive receptor LILRB4; and
(c) a peptide containing an amino acid sequence having 80% or more identity to the amino acid sequence represented by SEQ ID NO: 1, and having a binding capacity to a fibronectin binding site of the immunosuppressive receptor LILRB4.

[6] A therapeutic agent for an immune checkpoint-related disease containing a substance that inhibits binding between fibronectin and an immunosuppressive receptor LILRB4 as an active ingredient.
[7] The therapeutic agent according to [6], wherein the fibronectin binds to the immunosuppressive receptor LILRB4 via the amino acid sequence represented by SEQ ID NO: 1 in the fibronectin.
[8] The therapeutic agent according to [6] or [7], wherein the substance that inhibits binding between the fibronectin and the immunosuppressive receptor LILRB4 is an anti-fibronectin antibody or a derivative thereof, an anti-immunosuppressive receptor LILRB4 antibody or a derivative thereof, or a fibronectin analog.
[9] The therapeutic agent according to [8], wherein the anti-fibronectin antibody or a derivative thereof binds to the amino acid sequence represented by SEQ ID NO: 1 in the fibronectin.
[10] The therapeutic agent according to [8], wherein the fibronectin analog is any one of the following peptides (a) to (c):

(a) a peptide containing the amino acid sequence represented by SEQ ID NO: 1;
(b) a peptide containing an amino acid sequence in which one to several amino acids have been deleted, inserted, substituted, or added in the amino acid sequence represented by SEQ ID NO: 1, and having a binding capacity to a fibronectin binding site of the immunosuppressive receptor LILRB4; and
(c) a peptide containing an amino acid sequence having 80% or more identity to the amino acid sequence represented by SEQ ID NO: 1, and having a binding capacity to a fibronectin binding site of the immunosuppressive receptor LILRB4.

[11] The therapeutic agent according to any one of [6] to [10], wherein the immune checkpoint-related disease is selected from the group consisting of an autoimmune disease, a cancer, an inflammatory disease, and an allergic disease.

[12] The therapeutic agent according to [11], wherein the cancer is due to cancer metastasis.

[13] A therapeutic agent for an immune checkpoint-related disease containing a substance that activates an immunosuppressive receptor LILRB4 as an active ingredient.

[14] The therapeutic agent according to [13], wherein the substance that activates the immunosuppressive receptor LILRB4 is an anti-fibronectin antibody or a derivative thereof, an anti-immunosuppressive receptor LILRB4 antibody or a derivative thereof, or fibronectin or a fibronectin analog.

[15] The therapeutic agent according to [14], wherein the fibronectin analog is any one of the following peptides (a) to (c):

(a) a peptide containing the amino acid sequence represented by SEQ ID NO: 1;
(b) a peptide containing an amino acid sequence in which one to several amino acids have been deleted, inserted, substituted, or added in the amino acid sequence represented by SEQ ID NO: 1, and having a binding capacity to a fibronectin binding site of the immunosuppressive receptor LILRB4; and
(c) a peptide containing an amino acid sequence having 80% or more identity to the amino acid sequence represented by SEQ ID NO: 1, and having a binding capacity to a fibronectin binding site of the immunosuppressive receptor LILRB4.

[16] The therapeutic agent according to any one of [13] to [15], wherein the immune checkpoint-related disease is a bone disease.

[17] An immunosuppressant containing a substance that activates an immunosuppressive receptor LILRB4 as an active ingredient.

[18] The immunosuppressant according to [17], wherein the substance that activates the immunosuppressive receptor LILRB4 is an anti-fibronectin antibody or a derivative thereof, an anti-immunosuppressive receptor LILRB4 antibody or a derivative thereof, or fibronectin or a fibronectin analog.

[19] The immunosuppressant according to [18], wherein the fibronectin analog is any one of the following peptides (a) to (c):

(a) a peptide containing the amino acid sequence represented by SEQ ID NO: 1;
(b) a peptide containing an amino acid sequence in which one to several amino acids have been deleted, inserted, substituted, or added in the amino acid sequence represented by SEQ ID NO: 1, and having a binding capacity to a fibronectin binding site of the immunosuppressive receptor LILRB4; and
(c) a peptide containing an amino acid sequence having 80% or more identity to the amino acid sequence represented by SEQ ID NO: 1, and having a binding capacity to a fibronectin binding site of the immunosuppressive receptor LILRB4.

[20] An anti-fibronectin antibody or a derivative thereof, which binds to the amino acid sequence represented by SEQ ID NO: 1.

[21] A fibronectin analog, in which any one of the following peptides (a) to (c) and an Fc region of immunoglobulin G are fused with each other:

(a) a peptide containing the amino acid sequence represented by SEQ ID NO: 1;
(b) a peptide containing an amino acid sequence in which one to several amino acids have been deleted, inserted, substituted, or added in the amino acid sequence represented by SEQ ID NO: 1, and having a binding capacity to a fibronectin binding site of the immunosuppressive receptor LILRB4; and
(c) a peptide containing an amino acid sequence having 80% or more identity to the amino acid sequence represented by SEQ ID NO: 1, and having a binding capacity to a fibronectin binding site of the immunosuppressive receptor LILRB4.

[22] A kit for detecting fibronectin containing the amino acid sequence represented by SEQ ID NO: 1 or a partial protein thereof contained in a biological sample, including the anti-fibronectin antibody or a derivative thereof according to [20].

[23] A method for detecting fibronectin or a partial protein thereof in a biological sample using the kit according to [22].

Advantageous Effects of Invention

According to the present invention, an immune checkpoint inhibitor, a therapeutic agent for an immune checkpoint-related disease, an immunosuppressant, an anti-fibronectin antibody or a derivative thereof, a fibronectin analog, a kit for detecting fibronectin or a partial protein thereof, and a method for detecting fibronectin or a partial protein thereof can be provided.

BRIEF DESCRIPTION OF DRAWINGS

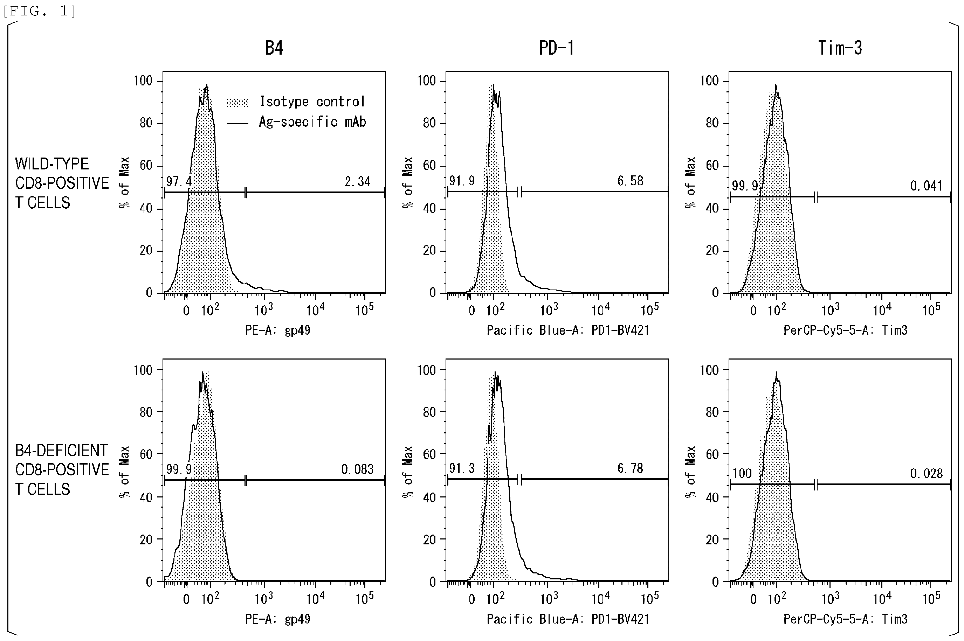

FIG. 1 shows the results of analyzing the expression of B4 (left), PD-1 (center), and Tim-3 (right) in mouse wild-type naive CD8-positive T cells (upper) and B4-deficient naive CD8-positive T cells (lower) by flow cytometry. The vertical axis represents the percentage of cells when the peak value of the histogram is assumed to be 100%, and the horizontal axis represents the fluorescence intensity (protein expression intensity). A solid line indicates the result of staining with each antigen-specific antibody, and gray without solid line indicates the result of staining with an isotype control antibody.

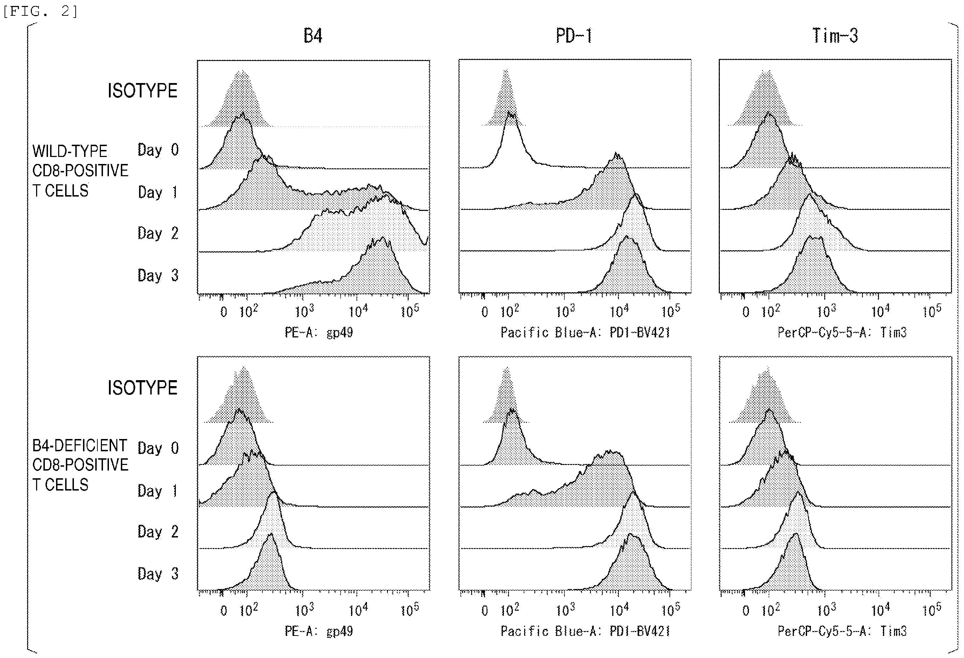

FIG. 2 shows the results of analyzing the expression of B4 (left), PD-1 (center), and Tim-3 (right) under activation stimulation with an anti-CD3 antibody/an anti-CD28 antibody in mouse wild-type CD8-positive T cells (upper) and B4-deficient CD8-positive T cells (lower) by flow cytometry over time (from day 0 to day 3). The results are shown as a graph of overlapping histograms on each measurement day, in which the vertical axis represents the percentage of cells when the peak value of the histogram is assumed to be 100%, and the horizontal axis represents the fluorescence intensity (protein expression intensity). A solid line indicates the result of staining with each antigen-specific antibody, and gray without solid line indicates the result of staining with an isotype control antibody.

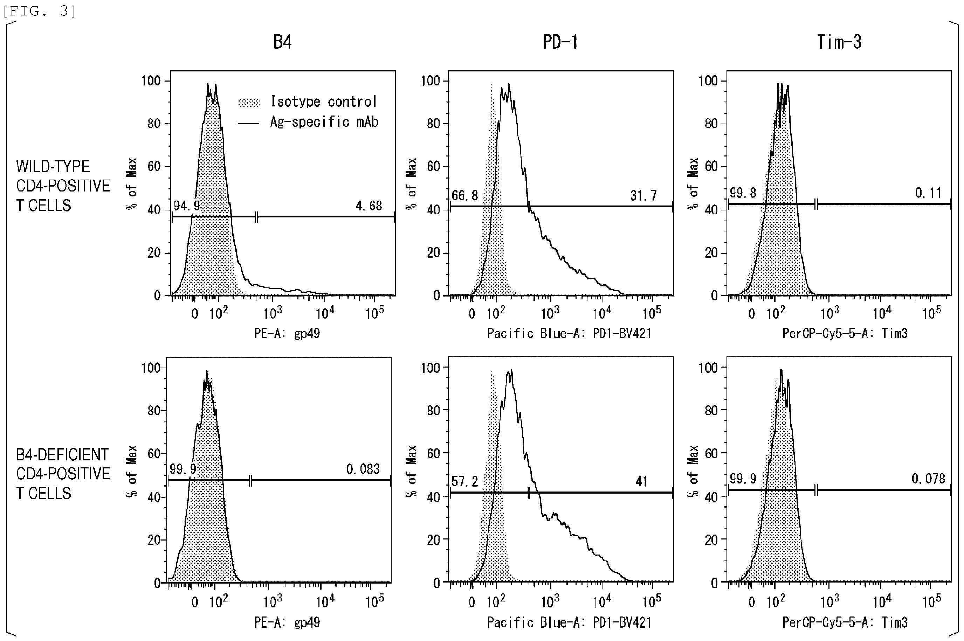

FIG. 3 shows the results of analyzing the expression of B4 (left), PD-1 (center), and Tim-3 (right) in mouse wild-type naive CD4-positive T cells (upper) and B4-deficient naive CD4-positive T cells (lower) by flow cytometry. The vertical axis represents the percentage of cells when the peak value of the histogram is assumed to be 100%, and the horizontal axis represents the fluorescence intensity (protein expression intensity). A solid line indicates the result of staining with each antigen-specific antibody, and gray without solid line indicates the result of staining with an isotype control antibody.

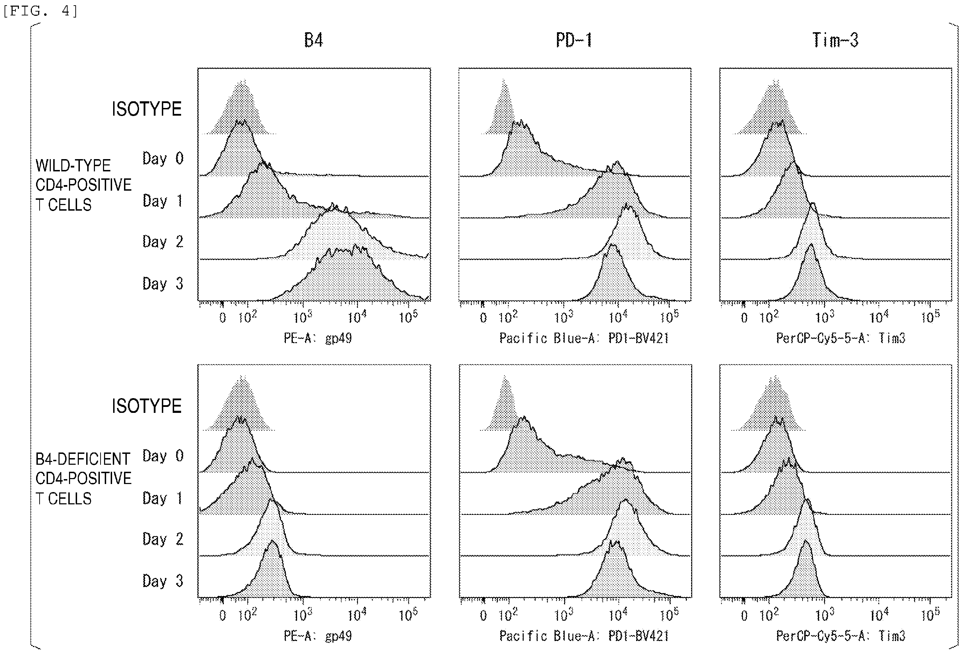

FIG. 4 shows the results of analyzing the expression of B4 (left), PD-1 (center), and Tim-3 (right) under activation stimulation with an anti-CD3 antibody/an anti-CD28 antibody in mouse wild-type CD4-positive T cells (upper) and B4-deficient CD4-positive T cells (lower) by flow cytometry over time (from day 0 to day 3). The results are shown as a graph of overlapping histograms on each measurement day, in which the vertical axis represents the percentage of cells when the peak value of the histogram is assumed to be 100%, and the horizontal axis represents the fluorescence intensity (protein expression intensity). A solid line indicates the result of staining with each antigen-specific antibody, and gray without solid line indicates the result of staining with an isotype control antibody.

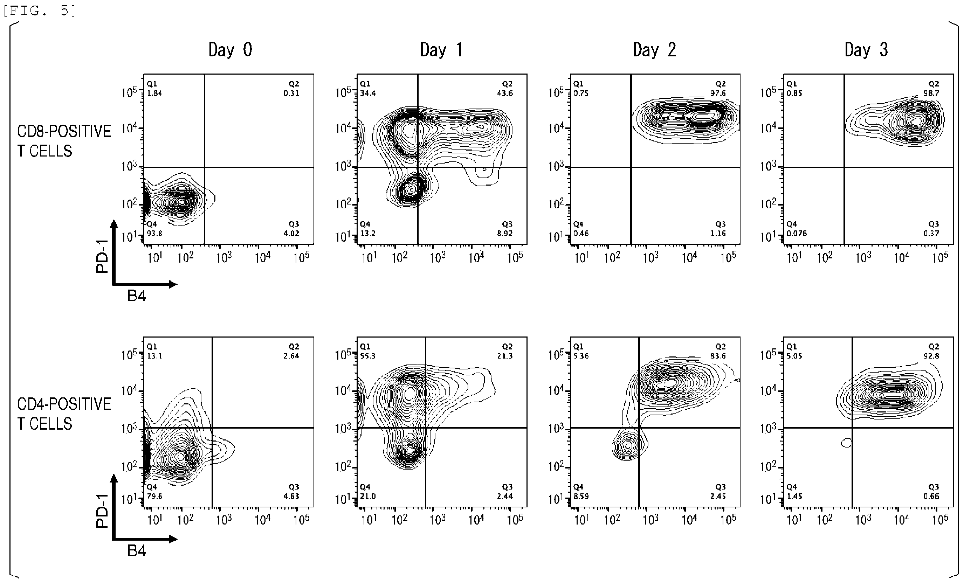

FIG. 5 shows the results of analyzing the coexpression of B4 and PD-1 under activation stimulation with an anti-CD3 antibody/an anti-CD28 antibody in mouse CD8-positive T cells (upper) and CD4-positive T cells (lower) by flow cytometry over time (from day 0 to day 3). The vertical axis represents the expression intensity of PD-1, the horizontal axis represents the expression intensity of B4, the lower left area separated by a cross indicates negative for PD-1 and B4, the upper left area indicates single positive for PD-1, the upper right area indicates positive for PD-1 and B4, and the lower right area indicates negative for PD-1 and positive for B4.

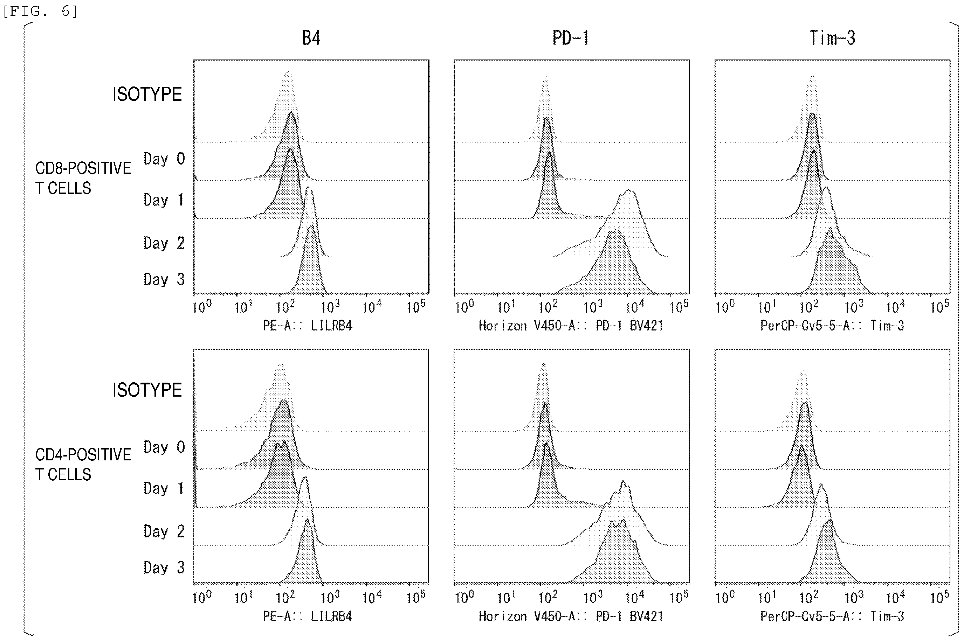

FIG. 6 shows the results of analyzing the expression of B4 (left), PD-1 (center), and Tim-3 (right) under activation stimulation with an anti-CD3 antibody/an anti-CD28 antibody in human CD8-positive T cells (upper) and CD4-positive T cells (lower) by flow cytometry over time (from day 0 to day 3). The results are shown as a graph of overlapping histograms on each measurement day, in which the vertical axis represents the percentage of cells when the peak value of the histogram is assumed to be 100%, and the horizontal axis represents the fluorescence intensity (protein expression intensity). A solid line indicates the result of staining with each antigen-specific antibody, and light gray indicates the result of staining with an isotype control antibody.

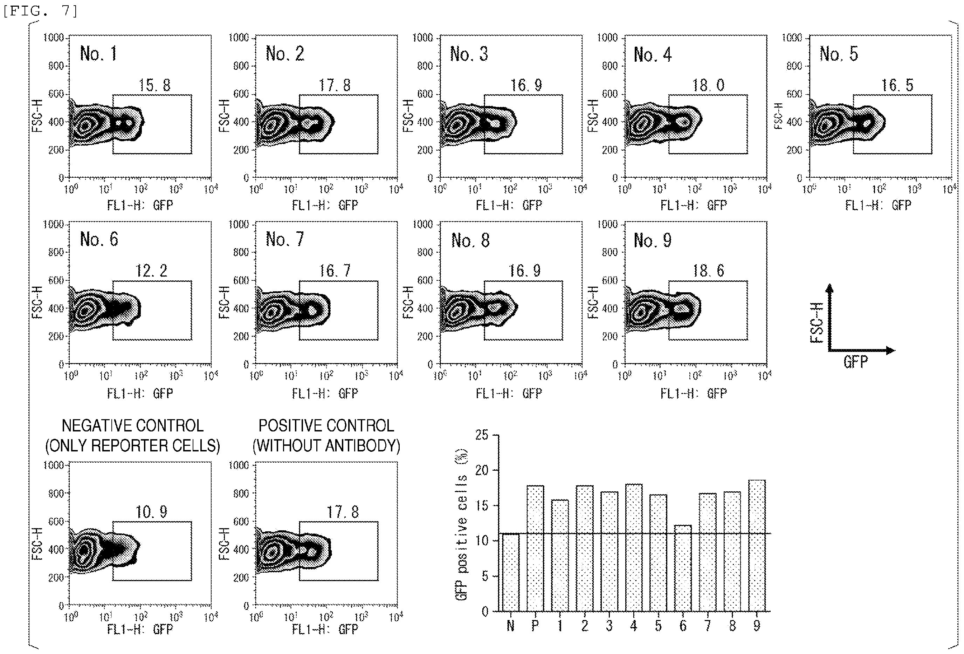

FIG. 7 shows the results of evaluating an FN-B4 binding inhibitory effect of an anti-FN30 monoclonal antibody in co-culture of human MSC and B4-GFP reporter cells based on the percentage of GFP-expressing cells of the B4-GFP reporter cells. The vertical axis in the plot represents the size of the cell, the horizontal axis represents the fluorescence intensity of GFP, and the numerical value in the plot indicates the percentage (%) of GFP-positive cells (in frame). The bar graph indicates the percentage (%) of GFP-positive cells when a treatment was performed with each antibody.

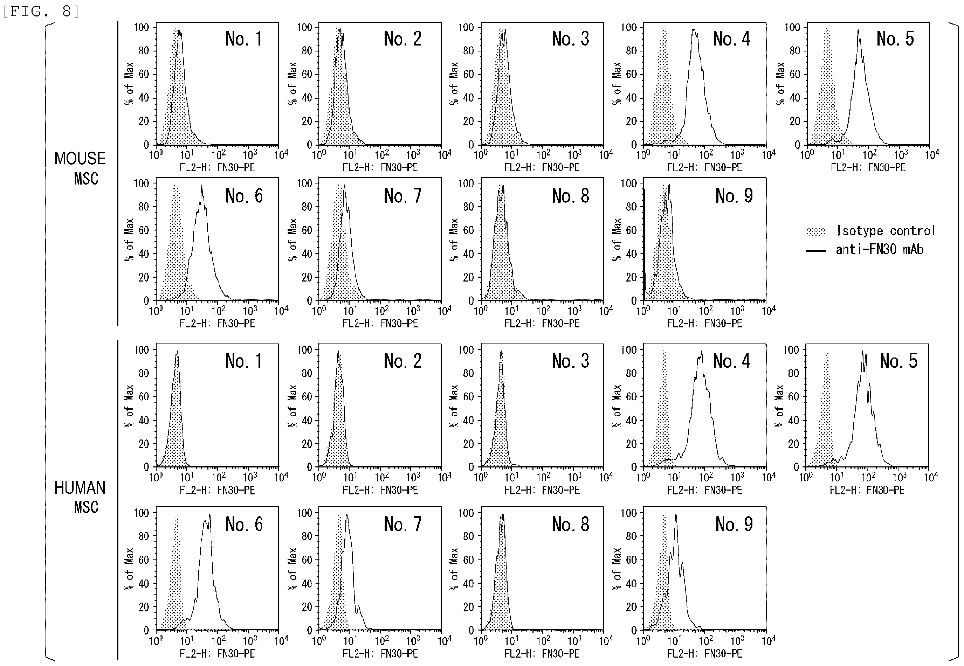

FIG. 8 shows the results of staining mouse bone marrow-derived mesenchymal stem cells and human bone marrow-derived mesenchymal stem cells with an anti-FN30 monoclonal antibody and performing an analysis using a flow cytometer. A solid line indicates the result of staining with the anti-FN30 monoclonal antibody, and gray without solid line indicates the result of staining with an isotype control antibody.

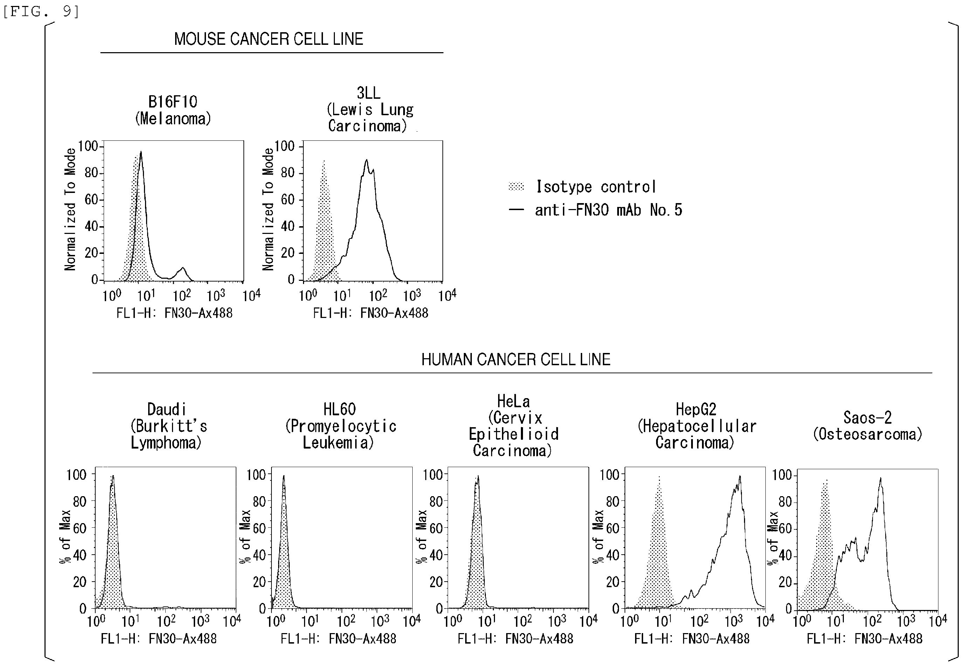

FIG. 9 shows the results of staining a mouse cancer cell line (upper) and a human cancer cell line (lower) using an anti-FN30 monoclonal antibody No. 5 and performing an analysis using a flow cytometer. A solid line indicates the result of staining with the anti-FN30 monoclonal antibody, and gray without solid line indicates the result of staining with an isotype control antibody.

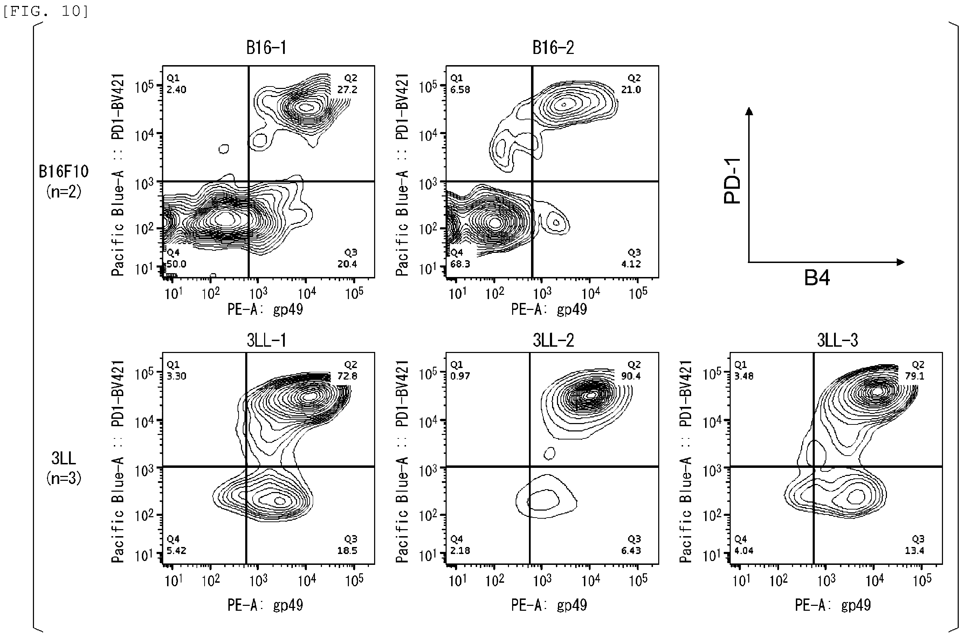

FIG. 10 shows the results of analyzing the expression of B4 and PD-1 in CD8-positive T cells infiltrated into a tumor formed by inoculating wild-type mice with a B16F10 cell line (upper, for two mice) and a 3LL cell line (lower, for three mice) with a flow cytometer. The vertical axis represents the expression intensity of PD-1, the horizontal axis represents the expression intensity of B4, the lower left area separated by a cross indicates negative for PD-1 and B4, the upper left area indicates single positive for PD-1, the upper right area indicates positive for PD-1 and B4, and the lower right area indicates negative for PD-1 and positive for B4.

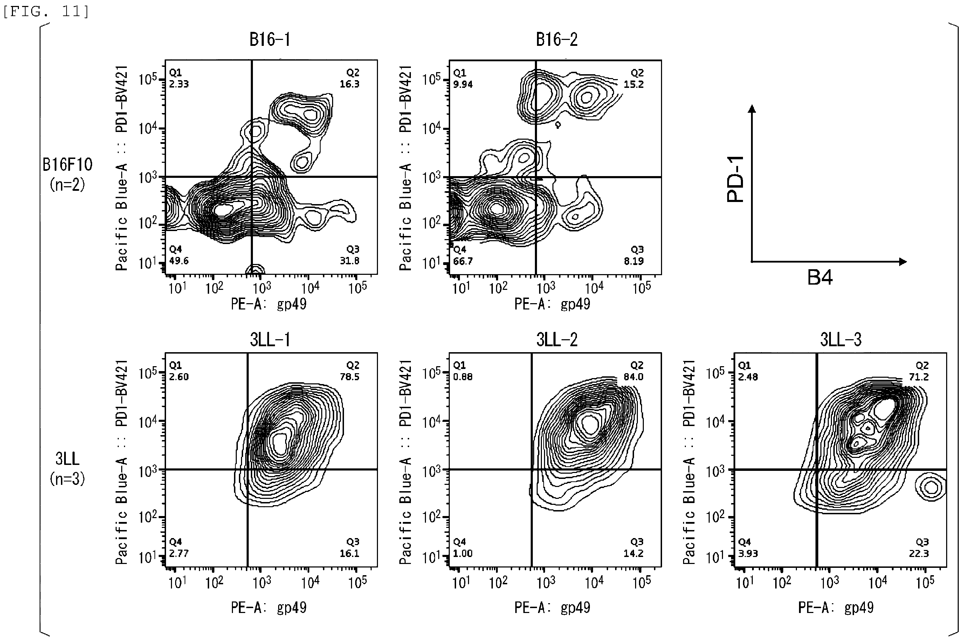

FIG. 11 shows the results of analyzing the expression of B4 and PD-1 in CD4-positive T cells infiltrated into a tumor formed by inoculating wild-type mice with a B16F10 cell line (upper, for two mice) and a 3LL cell line (lower, for three mice) with a flow cytometer. The vertical axis represents the expression intensity of PD-1, the horizontal axis represents the expression intensity of B4, the lower left area separated by a cross indicates negative for PD-1 and B4, the upper left area indicates single positive for PD-1, the upper right area indicates positive for PD-1 and B4, and the lower right area indicates negative for PD-1 and positive for B4.

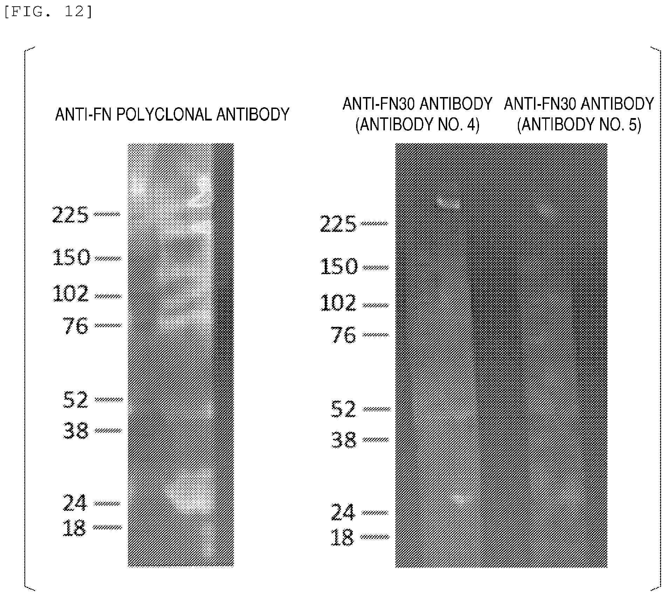

FIG. 12 shows the detection results of a protein by a Western blot method using an anti-fibronectin antibody in a human plasma sample. On the left, the result of staining using an anti-fibronectin polyclonal antibody is shown, and on the right, the results of staining using anti-FN30 monoclonal antibodies No. 4 and No. 5 are shown.

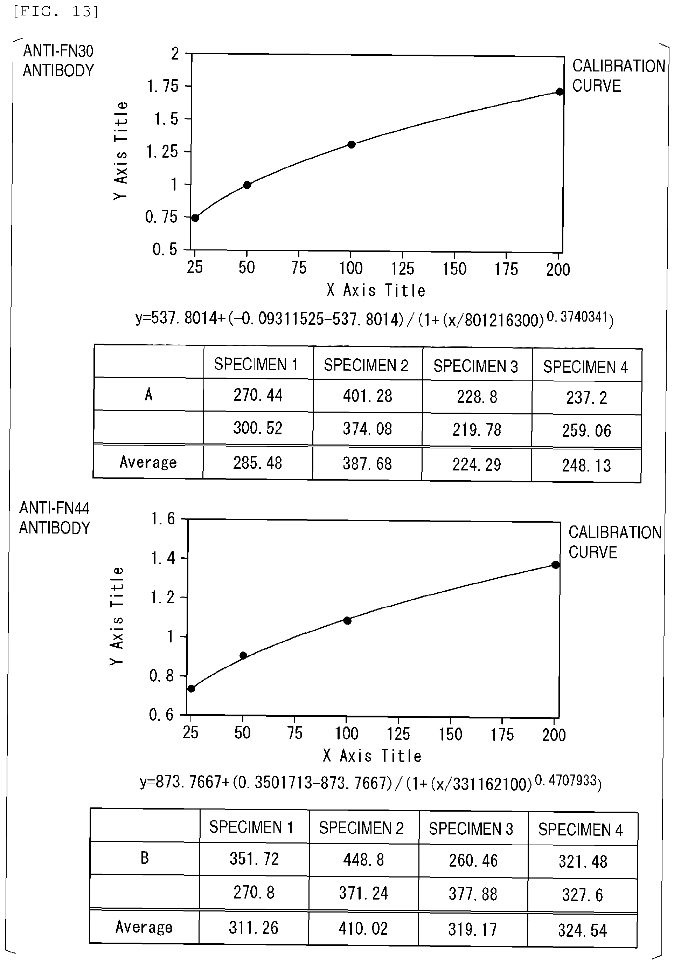

FIG. 13 shows the quantitative determination results of a full-length molecule of fibronectin and a 24 kDa fragment of fibronectin in healthy human plasma using an anti-fibronectin antibody. Nonlinear approximation was performed by 4-parameter logistic regression based on the absorbance of a standard protein, and the concentration was calculated from the absorbance value of the sample. In the upper part, the fibronectin concentration [A ($\mu$g/mL)] detected using an anti-FN30 monoclonal antibody is shown, and in the lower part, the fibronectin concentration [B ($\mu$g/mL)] detected using an anti-FN44 antibody is shown.

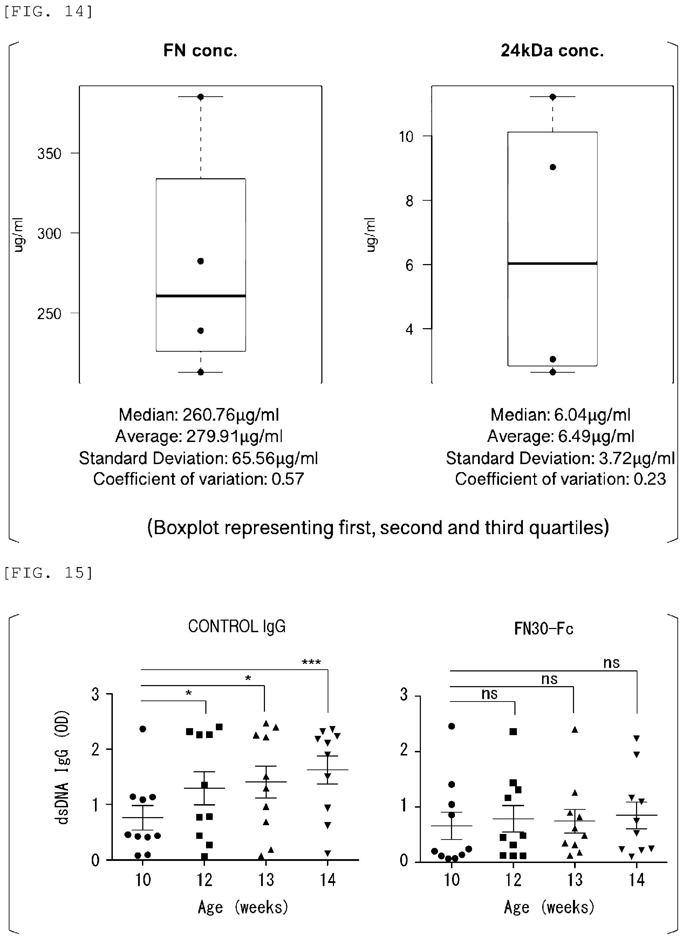

FIG. 14 shows boxplots representing the first, second, and third quartiles, and the maximum value and the minimum value of the concentrations of a full-length molecule of fibronectin and a 24 kDa fragment of fibronectin in healthy human plasma using an anti-FN monoclonal antibody. On the left, the concentration of the full-length molecule of fibronectin is shown, and on the right, the concentration of the 24 kDa fragment of fibronectin is shown.

FIG. 15 shows the results of intraperitoneally administering a control IgG or FN30-Fc to BXSB/Yaa mice and measuring an anti-dsDNA IgG level in serum.

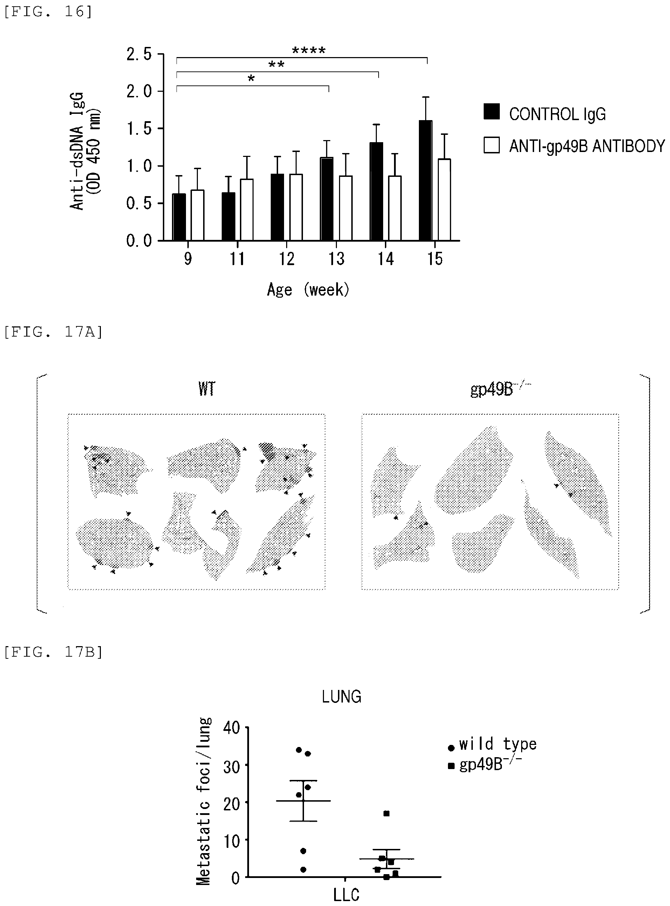

FIG. 16 shows the results of intraperitoneally administering a control IgG or an anti-gp49B monoclonal antibody H1.1 to BXSB/Yaa mice and measuring an anti-dsDNA IgG level in serum.

FIG. 17A shows the results of performing H & E staining of the lung surface 30 days after injecting Lewis lung carcinoma cells (LLC) into wild-type (WT) B6 mice and gp49B-deficient mice through the tail vein. WT shows the results of the wild-type mice, and gp49B$^{-/-}$ shows the results of the gp49B-deficient mice.

FIG. 17B shows the quantitative determination results of the number of metastatic foci in the lung 30 days after injecting Lewis lung carcinoma cells (LLC) into wild-type (WT) B6 mice and gp49B-deficient mice through the tail vein.

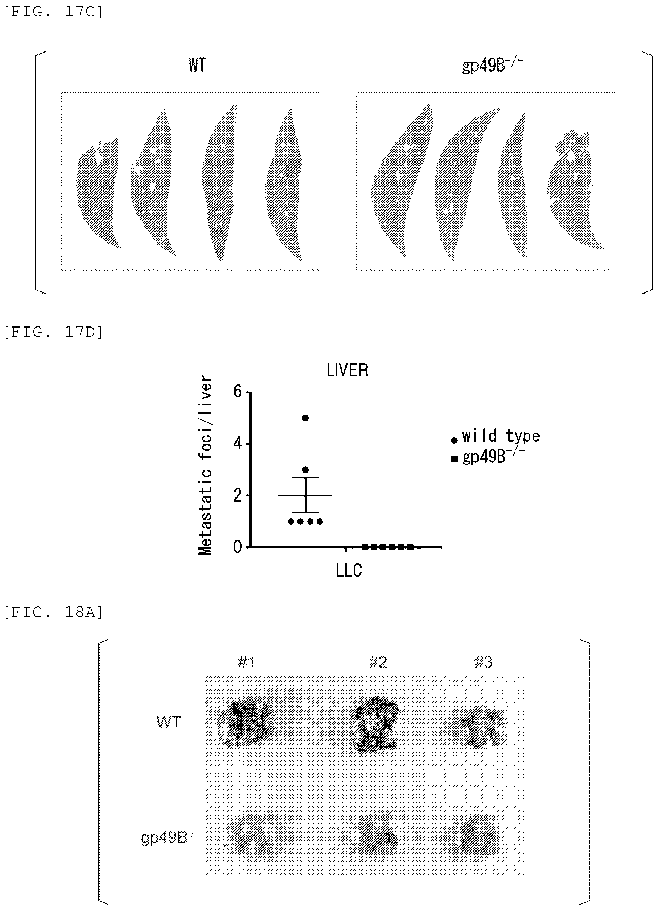

FIG. 17C shows the results of performing H & E staining of the liver 30 days after injecting Lewis lung carcinoma cells (LLC) into wild-type (WT) B6 mice and gp49B-deficient mice through the tail vein. WT shows the results of the wild-type mice, and gp49B$^{-/-}$ shows the results of the gp49B-deficient mice.

FIG. 17D shows the quantitative determination results of the number of metastatic foci in the liver 30 days after injecting Lewis lung carcinoma cells (LLC) into wild-type (WT) B6 mice and gp49B-deficient mice through the tail vein.

FIG. 18A shows the results of performing H & E staining of the lung surface 20 days after injecting mouse melanoma cells B16F10 into wild-type (WT) B6 mice and gp49B-deficient mice through the tail vein. WT denotes the wild-type mouse, and gp49B$^{-/-}$ denotes the gp49B-deficient mouse.

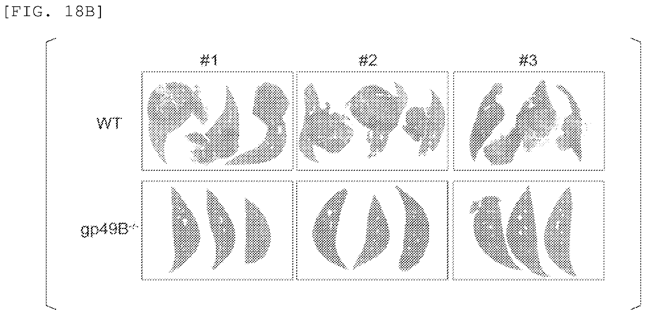

FIG. 18B shows the results of performing H & E staining of the liver 20 days after injecting mouse melanoma cells B16F10 into wild-type (WT) B6 mice and gp49B-deficient mice through the tail vein. WT denotes the wild-type mouse, and gp49B$^{-/-}$ denotes the gp49B-deficient mouse.

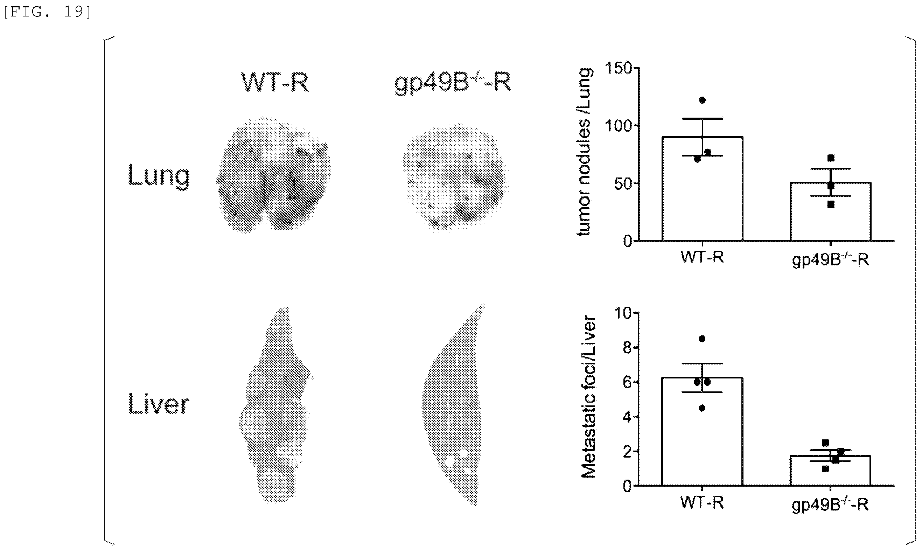

FIG. 19 shows the results of performing adoptive transfer of wild-type mouse bone marrow cells or gp49B-deficient mouse bone marrow cells. On the left, the results of H & E staining of the lung (upper) and the liver (lower) are shown, and on the right, the number of metastatic foci in the lung (upper) and in the liver (lower) is shown. WT-R denotes the wild-type mouse, and gp49B$^{-/-}$-R denotes the gp49B-deficient mouse.

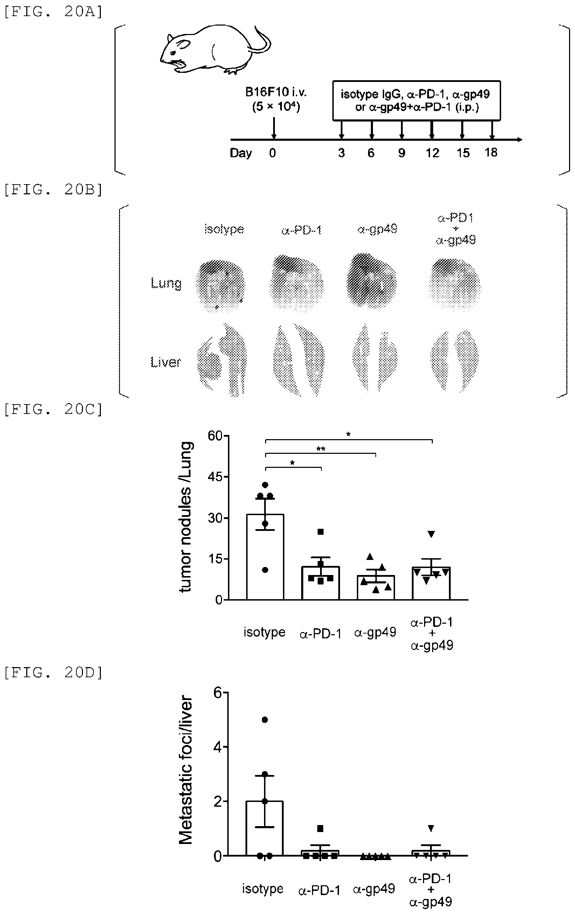

FIG. 20A shows a schedule of administering a control isotype IgG antibody, an anti-PD-1 monoclonal antibody, an anti-gp49B monoclonal antibody, or a combination of an anti-PD-1 monoclonal antibody and an anti-gp49B monoclonal antibody to wild-type (WT) B6 mice after injecting mouse melanoma cells B16F10 into the mice.

FIG. 20B is a view showing the metastatic states of the lung and the liver of mice in which a B16F10 tumor occurred by administering a control isotype IgG antibody, an anti-PD-1 monoclonal antibody, an anti-gp49B monoclonal antibody, or a combination of an anti-PD-1 monoclonal antibody and an anti-gp49B monoclonal antibody to wild-type (WT) B6 mice after injecting mouse melanoma cells B16F10 into the mice. In the upper part, lung tumor nodules are shown, and in the lower part, H & E-stained images of the liver are shown.

FIG. 20C is a graph of comparing the number of lung tumor nodules among the respective antibody-treated groups, in which a control isotype IgG antibody, an anti-PD-1 monoclonal antibody, an anti-gp49B monoclonal antibody, or a combination of an anti-PD-1 monoclonal antibody and an anti-gp49B monoclonal antibody was administered to wild-type (WT) B6 mice after injecting mouse melanoma cells B16F10 into the mice.

FIG. 20D is a graph of comparing the number of B16F10 metastatic foci in the liver among the respective antibody-treated groups in which a control isotype IgG antibody, an anti-PD-1 monoclonal antibody, an anti-gp49B monoclonal antibody, or a combination of an anti-PD-1 monoclonal antibody and an anti-gp49B monoclonal antibody was administered to wild-type (WT) B6 mice after injecting mouse melanoma cells B16F10 into the mice.

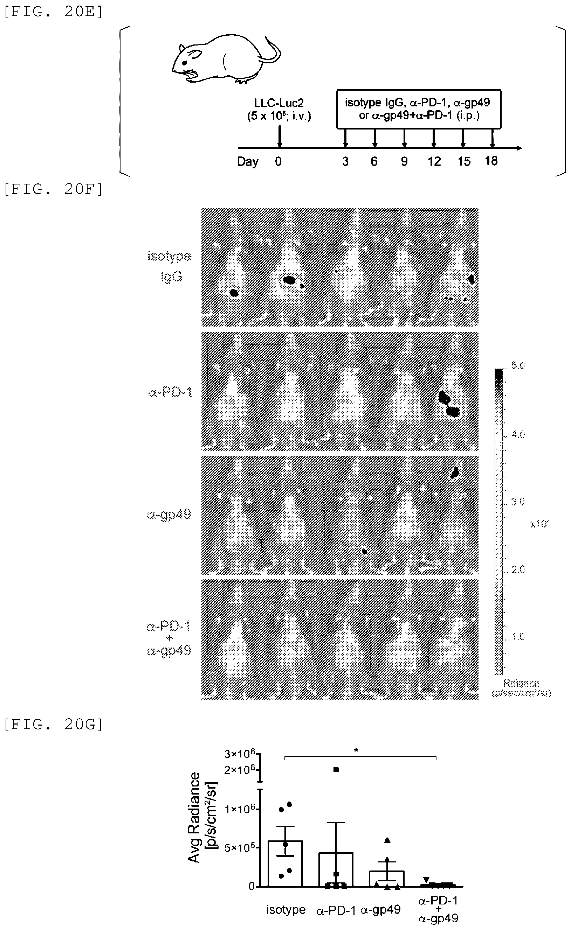

FIG. 20E shows a schedule of administering a control isotype IgG antibody, an anti-PD-1 monoclonal antibody, an anti-gp49B monoclonal antibody, or a combination of an anti-PD-1 monoclonal antibody and an anti-gp49B monoclonal antibody to wild-type (WT) B6 mice after injecting Lewis lung carcinoma cells (LLC) into the mice.

FIG. 20F shows the results of in vivo bioluminescence imaging of wild-type (WT) B6 mice in which Lewis lung carcinoma cells (LLC) were injected 21 days after administering a control isotype IgG antibody, an anti-PD-1 monoclonal antibody, an anti-gp49B monoclonal antibody, or a combination of an anti-PD-1 monoclonal antibody and an anti-gp49B monoclonal antibody to the mice after injecting the LLC cells into the mice.

FIG. 20G shows the results of performing a bioluminescence analysis with an average diameter (photons/s/cm$^2$/steradian) based on the images of in vivo bioluminescence imaging of wild-type (WT) B6 mice in which Lewis lung carcinoma cells (LLC) were injected 21 days after administering a control isotype IgG antibody, an anti-PD-1 monoclonal antibody, an anti-gp49B monoclonal antibody, or a combination of an anti-PD-1 monoclonal antibody and an anti-gp49B monoclonal antibody to the mice after injecting the LLC cells into the mice, and performing comparison among the respective antibody-treated groups.

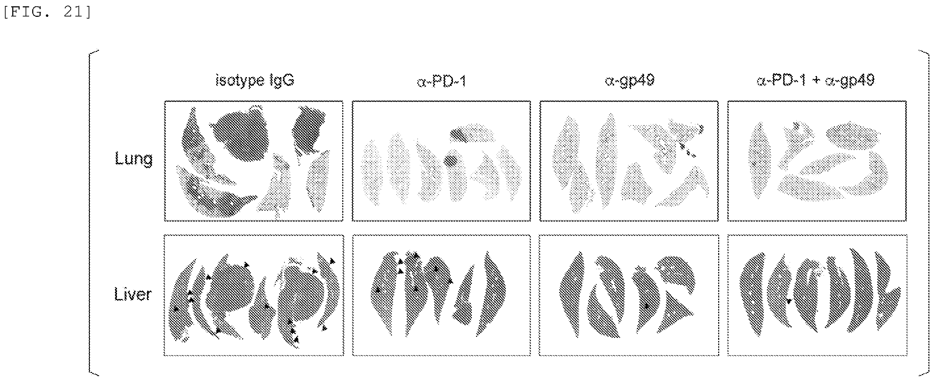

FIG. 21 shows the results of an analysis of pathological sections of the lung and the liver of wild-type (WT) B6 mice in which Lewis lung carcinoma cells (LLC) were injected, and thereafter a control isotype IgG antibody, an anti-PD-1 monoclonal antibody, an anti-gp49B monoclonal antibody, or a combination of an anti-PD-1 monoclonal antibody and an anti-gp49B monoclonal antibody was administered. In the upper part, H & E staining of the lung surface is shown, and in the lower part, H & E staining of the liver is shown. The arrows indicate the positions of cancer metastatic foci.

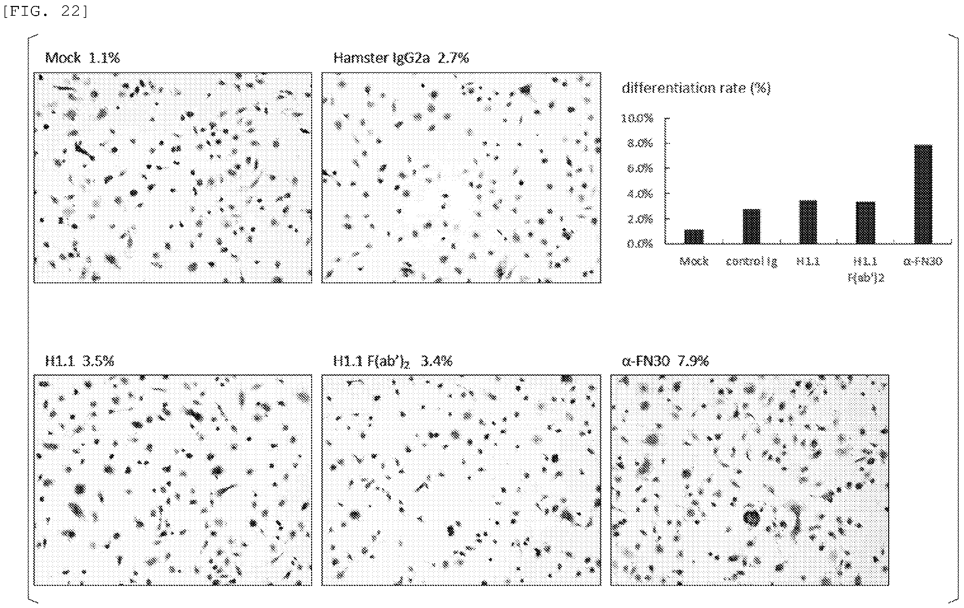

FIG. 22 shows the results of induction of differentiation from bone marrow cells into osteoclasts by adding a control isotype IgG antibody, an anti-gp49B monoclonal antibody H1.1, a F(ab')$_2$ fragment of the anti-gp49B monoclonal antibody H1.1, or an anti-FN30 monoclonal antibody No. 6. The photographs show TRAP-stained images of osteoclasts, and the graph shows the induction differentiation rate by the addition of the respective antibodies. In the photograph and the graph, Mock indicates a case where no antibody was added.

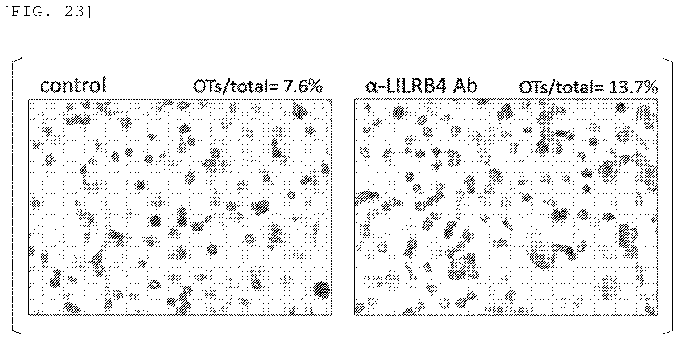

FIG. 23 shows TRAP-stained images of osteoclasts when an anti-LILRB4 monoclonal antibody ZM4.1 (right view) or mouse IgG1κ (left view) was added.

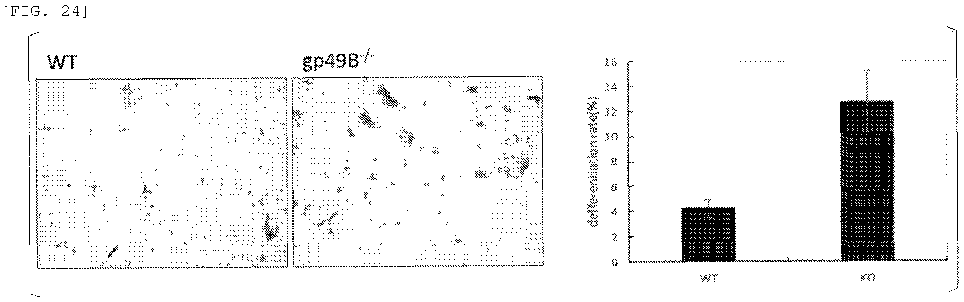

FIG. 24 shows the results of induction of differentiation into osteoclasts from bone marrow cells of a wild-type B6 mouse or a gp49B-deficient mouse. The left photograph shows a TRAP-stained image of osteoclasts differentiated from the bone marrow cells of the wild-type B6 mouse, and the right photograph shows a TRAP-stained image of osteoclasts differentiated from the bone marrow cells of the gp49B-deficient mouse. The graph shows the induction differentiation rates when differentiation into osteoclasts was induced from the bone marrow cells of the wild-type B6 mouse and the bone marrow cells of the gp49B-deficient mouse, respectively. WT denotes the wild-type B6 mouse, and gp49B$^{-/-}$ and KO denote the gp49B-deficient mouse.

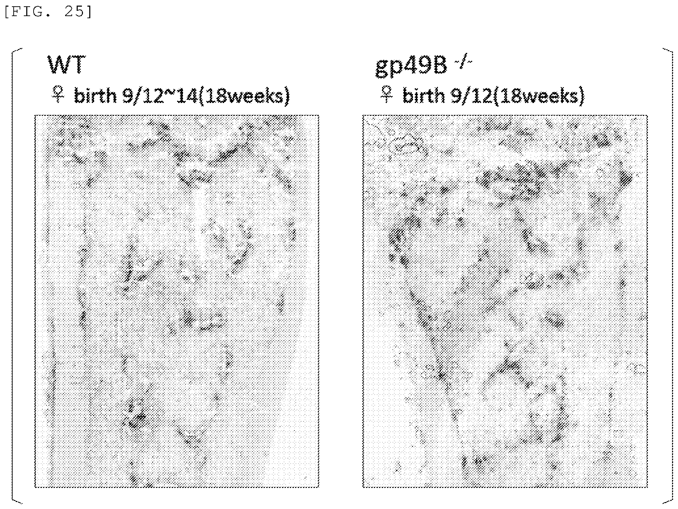

FIG. 25 shows TRAP-stained images of osteoclasts in the femurs of a wild-type B6 mouse and a gp49B-deficient mouse. The left view shows the TRAP-stained image of the osteoclasts of the wild-type B6 mouse and the right view shows the TRAP-stained image of the osteoclasts of the gp49B-deficient mouse.

## DESCRIPTION OF EMBODIMENTS

[Immune Checkpoint Inhibitor]

The immune checkpoint inhibitor of the present invention contains a substance that inhibits binding between fibronectin and an immunosuppressive receptor LILRB4 as an active ingredient. Fibronectin can bind to LILRB4 via the amino acid sequence represented by SEQ ID NO: 1 in the fibronectin. That is, the amino acid sequence represented by SEQ ID NO: 1 is a target sequence in the fibronectin of LILRB4.

The substance that inhibits binding between fibronectin and LILRB4 is not particularly limited as long as it is a substance having an activity of inhibiting binding between fibronectin and LILRB4, but an anti-fibronectin antibody or a derivative thereof, an anti-LILRB4 antibody or a derivative thereof, a fibronectin analog, or the like is exemplified.

The anti-fibronectin antibody may be either a monoclonal antibody or a polyclonal antibody as long as it is an antibody that reacts with fibronectin, but a monoclonal antibody is preferably used. The antibody can be produced by a well-known method. For example, in the production of a polyclonal antibody, a mouse, a rat, a hamster, a rabbit, a goat, a sheep, a chicken, or the like is used as an animal to be immunized. Antiserum can be obtained from serum after an antigen is administered under or into the skin, to the abdominal cavity, or the like of an animal once or multiple times. When a protein or a peptide is used as an antigen, immunization of a mixture with a replacement fluid having an immunostimulatory effect is more preferred.

Further, the monoclonal antibody can be produced according to a known monoclonal antibody production method, for example, "Monoclonal Antibody" co-written by Yoshiaki Nagamune and Hiroshi Terada, Hirokawa Shoten Co., Ltd. (1990), or "Monoclonal Antibody", Jame W. Golding, 3rd edition, Academic Press, 1996. In addition, a monoclonal antibody can also be produced by a DNA immunization method, and can be produced with reference to Nature 1992 Mar 12; 356 152-154 or J. Immunol Methods Mar 1; 249 147-154.

As the antigen used in the antibody production, fibronectin or a partial fragment (peptide) thereof, or a vector integrated with a cDNA encoding fibronectin or a partial fragment thereof can be used. In order to obtain a monoclonal antibody that inhibits binding between fibronectin and LILRB4, it is preferred to use a peptide containing the amino acid sequence represented by SEQ ID NO: 1, which is the target sequence of LILRB4 in the fibronectin. A fibronectin vector, which is a construct including a gene encoding a peptide containing the amino acid sequence represented by SEQ ID NO: 1, can be used as an optimal antigen gene for immunization. The DNA immunization method can be carried out by subcutaneously injecting the above-mentioned gene construct alone or in admixture into an animal (a mouse, a rat, or the like) so as to incorporate it into cells using any of various gene transfer methods (for example, intramuscular injection, electroporation, a gene gun, or the like) for an animal to be immunized.

The anti-fibronectin monoclonal antibody can be produced by a method in which a hybridoma prepared according to a conventional method is cultured, and the antibody is separated from the culture supernatant, or a method in which the hybridoma is administered to a mammal compatible with the hybridoma, and the antibody is collected as ascites. In addition, the anti-fibronectin monoclonal antibody can also be produced using a known gene recombination technique. Specifically, a monoclonal antibody to be produced by the hybridoma prepared above can be produced by cloning a gene encoding the antibody, preparing a vector containing the gene, transforming a host cell by introducing the vector into the host cell so as to obtain a cell that expresses the anti-fibronectin monoclonal antibody, and culturing the cell. The cell used in this preparation, the type of the vector, the type of the cell, the culture conditions, etc. are within the technical scope of those skilled in the art, and appropriate conditions can be set as appropriate.

The antibody can be used after purifying it as needed. Examples of the method for purifying and isolating the antibody include conventionally known methods, for example, salting out such as an ammonium sulfate precipitation method, a gel filtration method using Sephadex or the like, ion exchange chromatography, and an affinity purification method using a protein A column or the like.

Examples of the derivative of the anti-fibronectin antibody include $F(ab')_2$, $F(ab)_2$, Fab', Fab, Fv, and scFv of the anti-fibronectin monoclonal antibody, a mutant thereof, and a fusion protein or a fusion peptide containing an antibody moiety. The derivative of the anti-fibronectin antibody can be produced according to a known method for producing an antibody derivative.

The anti-fibronectin antibody or a derivative thereof binds to the amino acid sequence represented by SEQ ID NO: 1 in fibronectin or a partial sequence thereof. In the immune checkpoint inhibitor of the present invention, the anti-fibronectin antibody or a derivative thereof can inhibit binding between fibronectin and LILRB4 by binding to the amino acid sequence represented by SEQ ID NO: 1 in the fibronectin or a partial sequence thereof.

The anti-LILRB4 antibody may be either a monoclonal antibody or a polyclonal antibody as long as it is an antibody that binds to LILRB4, but a monoclonal antibody is preferably used. The anti-LILRB4 antibody can be produced in the same manner as the anti-fibronectin antibody.

As the antigen used in the anti-LILRB4 antibody production, LILRB4 protein or a partial fragment (peptide) thereof, or a vector integrated with a cDNA encoding LILRB4 protein can be used. In order to obtain a monoclonal antibody that recognizes the conformation of LILRB4, a full-length LILRB4 vector that is a construct including a human LILRB4 full-length gene is an optimal antigen gene for immunization, however, in addition thereto, a construct in which a partial region of the LILRB4 sequence is inserted can also be used as the antigen gene for immunization. As the partial region of the LILRB4 sequence, a region of LILRB4 in which the amino acid sequence represented by SEQ ID NO: 1 that is the target sequence of LILRB4 in fibronectin binds to LILRB4 (fibronectin binding site) is preferred. The DNA immunization method can be carried out by subcutaneously injecting the above-mentioned gene construct alone or in admixture into an animal (a mouse, a rat, or the like) so as to incorporate it into cells using any of various gene transfer methods (for example, intramuscular injection, electroporation, a gene gun, or the like) for an animal to be immunized.

Purification of the anti-LILRB4 antibody can be carried out in the same manner as that of the anti-fibronectin antibody. Examples of the derivative of the anti-LILRB4 antibody include $F(ab')_2$, $F(ab)_2$, Fab', Fab, Fv, and scFv of the anti-LILRB4 antibody, a mutant thereof, and a fusion protein or a fusion peptide containing an antibody moiety.

In the immune checkpoint inhibitor of the present invention, the anti-LILRB4 antibody or a derivative thereof can inhibit binding of fibronectin to LILRB4 via the amino acid sequence represented by SEQ ID NO: 1 in the fibronectin.

Measurement of the inhibition of binding between fibronectin and LILRB4 can be carried out by evaluating inhibition of binding of fibronectin to a cell that expresses LILRB4. The cell that expresses LILRB4 is not particularly limited as long as it is a cell that expresses LILRB4, but for example, spleen cells, peripheral blood leukocytes, bone marrow cells, or B cells isolated from them, plasma cells, monocytes or macrophages, dendritic cells, eosinophils, basophils, neutrophils, mast cells, activated T cells, and the like are exemplified.

The base sequence and the amino acid sequence of LILRB4 can be known from the database provided by the National Center for Biotechnology Information (NCBI). In the case of human (Homo sapiens) LILRB4, for example, Entrez Gene ID: 11006 (as of June 17, 2019), and RefSeq Protein ID: NP_001265355.2, NP_001265356.2, NP_001265357.2, NP_001265358.2, and NP_001265359.2, (corresponding to isoforms 1 to 5) are exemplified. As mouse (Mus musculus) LILRB4, Gene ID: 14728 (as of June 24, 2016) and NP_038560.1 are exemplified, and as rat (Rattus norvegicus) LILRB4, Gene ID: 292594 (as of April 18, 2019) and RefSeq Protein ID: NP_001013916 are exemplified, and it is known that other animals also have LILRB4. The present invention is not limited to the above-mentioned LILRB4, and other LILRB4 is also included in the LILRB4 in the present invention.

The base sequence and the amino acid sequence of fibronectin can be known from the database provided by the National Center for Biotechnology Information (NCBI). In the case of human (Homo sapiens) fibronectin, for example, Entrez Gene ID: 2335, RefSeq Protein ID: NP_997647, NP_001352447, XP_005246463, and the like are exemplified. As mouse (Mus musculus) fibronectin, Gene ID: 14268 is exemplified, and as rat (Rattus norvegicus) fibronectin, Gene ID: 25661 and RefSeq Protein ID: NP_062016 are exemplified, and it is known that other animals also have fibronectin. The present invention is not limited to the above-mentioned fibronectins, and other fibronectins are also included in the fibronectin in the present invention.

Fibronectin binds to LILRB4 present on the cell surface of a plasma cell, a T cell, a macrophage, or the like via the amino acid sequence represented by SEQ ID NO: 1 in the fibronectin, and activates an immune checkpoint molecule. In other words, LILRB4 exhibits an immunosuppressive function by binding to fibronectin via the amino acid sequence represented by SEQ ID NO: 1 in the fibronectin.

In the present invention, the fibronectin analog includes any fibronectin analog as long as it has an action of inhibiting binding between fibronectin and LILRB4, and for example, any one of the following peptides (a) to (c) is exemplified:

(a) a peptide containing the amino acid sequence represented by SEQ ID NO: 1;
(b) a peptide containing an amino acid sequence in which one to several amino acids have been deleted, inserted, substituted, or added in the amino acid sequence represented by SEQ ID NO: 1, and having a binding capacity to a fibronectin binding site of the immunosuppressive receptor LILRB4; and
(c) a peptide containing an amino acid sequence having 80% or more identity to the amino acid sequence represented by SEQ ID NO: 1, and having a binding capacity to a fibronectin binding site of the immunosuppressive receptor LILRB4.

The identity of the amino acid sequence is 80% or more, but is preferably 85% or more, more preferably 90% or more, further more preferably 95% or more, and still further more preferably 98% or more. In addition, the number of deletions, substitutions, or additions in the amino acid sequence is preferably 1 to 5, more preferably 1 to 4, further more preferably 1 to 3, and still further more preferably 1 to 2. The identity of the amino acid sequence can be determined by BLAST search provided on the GenBank database.

The fibronectin analog may be a fibronectin analog in which any one of the above peptides (a) to (c) and an Fc region of immunoglobulin G are fused with each other.

The fibronectin analog can be produced by a known method, for example, a gene recombination technique.

The immune checkpoint inhibitor of the present invention contains a substance that inhibits binding between fibronectin and LILRB4 as an active ingredient, and may further contain a pharmaceutically acceptable carrier or additive.

Examples of the carrier and the additive include water, physiological saline, a phosphate buffer solution, dextrose, glycerol, a pharmaceutically acceptable organic solvent such as ethanol, collagen, polyvinyl alcohol, polyvinylpyrrolidone, a carboxy vinyl polymer, sodium carboxymethyl cellulose, sodium polyacrylate, sodium alginate, water-soluble dextran, sodium carboxymethyl starch, pectin, methyl cellulose, ethyl cellulose, xanthan gum, gum Arabic, casein, agar, polyethylene glycol, diglycerin, glycerin, propylene glycol, vaseline, paraffin, stearyl alcohol , stearic acid, human serum albumin, mannitol, sorbitol, lactose, and a surfactant, but are not limited thereto.

The immune checkpoint inhibitor of the present invention can be formed into various forms, for example, a liquid (for example, an injection), a dispersion, a suspension, a tablet, a pill, a powder, a suppository, and the like. A preferred embodiment is an injection, which is preferably administered parenterally (for example, intravenously, transdermally, intraperitoneally, or intramuscularly).

The immune checkpoint inhibitor of the present invention can be used as a therapeutic agent for an immune checkpoint-related disease.

The dose of the immune checkpoint inhibitor of the present invention is, for example, from 0.025 to 50 mg/kg, preferably from 0.1 to 50 mg/kg, and more preferably can be set to 0.1 to 25 mg/kg, and further more preferably 0.1 to 10 mg/kg or 0.1 to 3 mg/kg, but is not limited thereto.

[Therapeutic Agent for Immune Checkpoint-Related Disease]

The therapeutic agent for an immune checkpoint-related disease of the present invention contains a substance that inhibits binding between fibronectin and LILRB4 as an active ingredient.

The substance that inhibits binding between fibronectin and LILRB4 is not particularly limited as long as it is a substance having an activity of inhibiting binding between fibronectin and LILRB4, but an anti-fibronectin antibody or a derivative thereof, an anti-LILRB4 antibody or a derivative thereof, a fibronectin analog, or the like is exemplified. As the anti-fibronectin antibody or a derivative thereof, the anti-LILRB4 antibody or a derivative thereof, and the fibronectin analog, those described above are exemplified.

In the present invention, the immune checkpoint-related disease is not particularly limited as long as it is a disease in which LILRB4 that is an immune checkpoint molecule is involved, but for example, an autoimmune disease, a cancer, an inflammatory disease, an allergic disease, and the like are exemplified.

Examples of the autoimmune diseases include Basedow's disease, rheumatoid arthritis, Hashimoto thyroiditis, type 1 diabetes, systemic lupus erythematosus, vasculitis, Addison's disease, polymyositis, dermatomyositis, psoriasis, Sjogren's syndrome, systemic scleroderma, and glomerulonephritis.

Examples of the cancer include lung cancer, colon cancer, renal cancer, malignant melanoma, Hodgkin's lymphoma, head and neck cancer, pancreatic cancer, liver cancer, prostate cancer, osteosarcoma, and leukemia. The cancer may be primary or metastatic, however, the therapeutic agent is preferably used for a metastatic cancer.

Examples of the inflammatory disease include systemic lupus erythematosus, dermatomyositis, Kawasaki disease, psoriasis, herpes zoster, chronic obstructive pulmonary disease (COPD), bronchial asthma, atopic dermatitis, rheumatoid arthritis, antiphospholipid antibody syndrome, polymyositis, vasculitic syndrome, Sjogren's syndrome, Behcet's disease, Basedow's disease, Hashimoto's disease, myocarditis, aortitis syndrome, ulcerative colitis, Crohn's disease, primary biliary cirrhosis, autoimmune hepatitis, autoimmune pancreatitis, multiple sclerosis, myasthenia gravis, Guillain-Barre syndrome, glomerulonephritis, ANCA-associated nephritis, amyloidosis, TINU syndrome, irritable pneumonia, eosinophilic pneumonia, and sarcoidosis.

Examples of the allergic disease include allergic rhinitis, bronchial asthma, urticaria/atopic dermatitis, herpes zoster, chronic obstructive pulmonary disease (COPD), allergic conjunctivitis, food allergy, anaphylaxis, autoimmune hemolytic anemia, thrombocytopenia, granulocytopenia, neonatal hemolytic jaundice, serum sickness, hypersensitivity pneumonitis, lupus nephritis (chronic glomerulonephritis), systemic lupus erythematosus, contact dermatitis, Hashimoto's disease, Behcet's disease, and rejection reaction or graft-versus-host disease (GVHD) after organ transplantation.

The therapeutic agent for an immune checkpoint-related disease of the present invention may contain a substance that activates LILRB4 as an active ingredient. The substance that activates LILRB4 is not particularly limited as long as it is a substance that activates LILRB4 by binding to LILRB4, but for example, an anti-fibronectin antibody or a derivative thereof, an anti-LILRB4 antibody or a derivative thereof, fibronectin or a fibronectin analog, or the like is exemplified. As the anti-fibronectin antibody or a derivative thereof, the anti-LILRB4 antibody or a derivative thereof, and the fibronectin or the fibronectin analog, those described above are exemplified. However, in the therapeutic agent for an immune checkpoint-related disease containing a substance that activates LILRB4 as an active ingredient of the present invention, the anti-fibronectin antibody or a derivative thereof, the anti-LILRB4 antibody or a derivative thereof, and the fibronectin or the fibronectin analog activate LILRB4 by binding to LILRB4 so as to exhibit the immunosuppressive function of LILRB4.

In the therapeutic agent for an immune checkpoint-related disease containing a substance that activates LILRB4 as an active ingredient of the present invention, the immune checkpoint-related disease is not particularly limited as long as it is a disease on which the therapeutic agent has a therapeutic effect by activating LILRB4 so as to suppress an immune function, but for example, a bone disease such as rheumatoid arthritis, marble bone disease, or osteoporosis, or the like is exemplified. The substance that activates LILRB4 of the present invention, for example, can treat a bone disease by suppressing proliferation of osteoclasts.

The therapeutic agent for an immune checkpoint-related disease of the present invention may further contain a pharmaceutically acceptable carrier or additive. As the pharmaceutically acceptable carrier and additive, those described above are exemplified.

The therapeutic agent for an immune checkpoint-related disease of the present invention can be formed into various forms, for example, a liquid (for example, an injection), a dispersion, a suspension, a tablet, a pill, a powder, a suppository, and the like. A preferred embodiment is an injection, which is preferably administered parenterally (for example, intravenously, transdermally, intraperitoneally, or intramuscularly).

The dose of the therapeutic agent for an immune checkpoint-related disease of the present invention is, for example, from 0.025 to 50 mg/kg, preferably from 0.1 to 50 mg/kg, and more preferably can be set to 0.1 to 25 mg/kg, and further more preferably 0.1 to 10 mg/kg or 0.1 to 3 mg/kg, but is not limited thereto.

[Immunosuppressant]

The immunosuppressant of the present invention contains a substance that activates LILRB4 as an active ingredient. As the substance that activates LILRB4, those exemplified for the therapeutic agent for an immune checkpoint-related disease are exemplified. In the immunosuppressant containing a substance that activates LILRB4 as an active ingredient of the present invention, an anti-fibronectin antibody or a derivative thereof, an anti-LILRB4 antibody or a derivative thereof, or fibronectin or a fibronectin analog activates LILRB4 by binding to LILRB4 so as to exhibit the immunosuppressive function of LILRB4. The immunosuppressant of the present invention can be applied to transplantation therapy.

The immunosuppressant of the present invention may further contain a pharmaceutically acceptable carrier or additive. As the pharmaceutically acceptable carrier and additive, those described above are exemplified.

The immunosuppressant of the present invention can be formed into various forms, for example, a liquid (for example, an injection), a dispersion, a suspension, a tablet, a pill, a powder, a suppository, and the like. A preferred embodiment is an injection, which is preferably administered parenterally (for example, intravenously, transdermally, intraperitoneally, or intramuscularly).

The dose of the immunosuppressant of the present invention is, for example, from 0.025 to 50 mg/kg, preferably from 0.1 to 50 mg/kg, and more preferably can be set to 0.1 to 25 mg/kg, and further more preferably 0.1 to 10 mg/kg or 0.1 to 3 mg/kg, but is not limited thereto.

[Fibronectin Detection Kit and Fibronectin Detection Method]

The kit for detecting fibronectin or a partial peptide thereof of the present invention includes an anti-fibronectin antibody or a derivative thereof that binds to the amino acid sequence represented by SEQ ID NO: 1, and can detect the fibronectin or a partial peptide thereof in a biological sample.

The biological sample is not particularly limited, and examples thereof include blood, saliva, urine, spinal fluid, bone marrow aspirate, pleural effusion, ascites, joint fluid, tear fluid, aqueous humor, vitreous humor, and lymph fluid.

In the kit of the present invention, the partial peptide of fibronectin is not particularly limited as long as it binds to an anti-fibronectin antibody or a derivative thereof, but is preferably a partial peptide containing the amino acid sequence represented by SEQ ID NO: 1 and having a molecular weight of 24 kDa.

In the kit of the present invention, in addition to the anti-fibronectin antibody or a derivative thereof that binds to the amino acid sequence represented by SEQ ID NO: 1, another constituent element necessary for the detection of fibronectin, for example, a reaction buffer solution or a reaction vessel can be included.

By using the kit for detecting fibronectin or a partial peptide thereof of the present invention, fibronectin or a partial peptide thereof in a biological sample can be detected.

The method for detecting fibronectin or a partial peptide thereof in a biological sample is not particularly limited as long as an anti-fibronectin antibody or a derivative thereof that binds to the amino acid sequence represented by SEQ ID NO: 1 is included, and fibronectin or a partial peptide thereof in a biological sample can be detected, but it is preferred to perform measurement by an immunological method using the anti-fibronectin antibody or a derivative thereof. As the immunological method, for example, immunostaining (Western blot), an enzyme-linked immunosorbent assay (ELISA), sandwich ELISA, immunoprecipitation, immunoturbidimetry (TIA or LTIA), an enzyme immunoassay, a chemiluminescent immunoassay, a fluorescent immunoassay, a flow cytometry method, or the like is used, and it can be carried out by detecting a band or a spot or a peak that is in agreement with the molecular weight of fibronectin or a partial peptide thereof, but the method is not limited thereto.

Examples

Next, the present invention will be described in more detail with reference to Examples, but the present invention is not limited to the following Examples.

[Example 1] Expression of LILRB4 in Mouse T Cells

To a 48-well plate (manufactured by Thermo Fisher Scientific, Inc., #150687), 100 μL of a PBS(-) buffer solution containing each of an anti-mouse CD3 antibody (manufactured by BD Biosciences, Inc., Clone: 145-2C11, #553058) and an anti-mouse CD28 antibody (manufactured by BD Biosciences, Inc., Clone: 37.51, #553298) at a concentration of 10 pg/mL was added to coat the wells therewith at room temperature for 30 minutes, followed by washing the wells three times with 500 μL of a PBS(-) buffer solution. Spleen cells collected from the spleen of a 10 week old female gp49B-deficient mouse or wild-type mouse were subjected to a hemolytic treatment, and thereafter suspended in RPMI-1640 medium (manufactured by Sigma-Aldrich Co. LLC, #R8758) containing 10% fetal bovine serum (manufactured by BioWest, #S1530), 50 μM 2-mercaptoethanol (manufactured by FUJIFILM Wako Pure Chemical Corporation, #139-06861), and a 1% penicillin (5000 U/mL)/streptomycin (5000 μg/mL) solution (manufactured by Sigma-Aldrich Co. LLC, #P4458). Note that gp49B is a molecule homologous to human LILRB4 in mice.

In the wells coated with the antibody, the suspended spleen cells were seeded at a concentration of $1 \times 10^6$ cells/200 μL/well and cultured at 37°C under 5% carbon dioxide conditions for 0 to 3 days. The cells were recovered from the wells, and thereafter suspended in a PBS(-) buffer solution containing 2% fetal bovine serum and 0.05% sodium azide (manufactured by Sigma-Aldrich Co. LLC, #S8032) and stained under ice temperature conditions using an FITC-labeled anti-mouse CD4 antibody (manufactured by BioLegend, Clone: GK1.5, #100406), an Alexa 647-labeled anti-mouse CD8a antibody (manufactured by BD Biosciences, Inc., Clone: 53-6.7, #557882), a PE-labeled anti-mouse gp49A/B antibody (manufactured by BioLegend, Clone: H1.1, #144904), a BV421-labeled anti-mouse PD-1 antibody (manufactured by BioLegend, Clone: 29F.1A12, #135218), or PerCP-Cy5.5-labeled anti-mouse Tim-3 antibody (manufactured by BioLegend, Clone: B8.2C12, #134012), or as an isotype control antibody, PE-labeled Armenian hamster IgG (manufactured by BioLegend, Clone: HTK888, #400908), BV421-labeled rat IgG2a (manufactured by BioLegend, Clone: RTK2758, #400549), or PerCP-Cy5.5-labeled rat IgG1 (manufactured by BioLegend, Clone: RTK2071, #400426), and measured by BD FACS Aria III cell sorter (manufactured by BD Biosciences, Inc.), and data were analyzed by FlowJo software (manufactured by BD Biosciences, Inc.). The results are shown in FIGS. 1 to 5.

In FIG. 1, the expression of B4, PD-1, and Tim-3 in mouse naive CD8-positive T cells is shown. As shown in FIG. 1, in the mouse naive CD8-positive T cells, the expression of PD-1 was slightly observed, but the expression of B4 and Tim-3 was not observed. In addition, the effect on the expression of PD-1 or Tim-3 by B4 deletion was not observed.

In FIG. 2, the expression of B4, PD-1, and Tim-3 after stimulation with the anti-CD3 antibody/the anti-CD28 antibody in mouse CD8-positive T cells is shown. As shown in FIG. 2, by activation stimulation with the anti-CD3 antibody/the anti-CD28 antibody, the expression of PD-1 on the mouse CD8-positive T cells was almost maximized on day 1, but the expression of B4 and Tim-3 slightly increased on day 1, and was maximized on day 2 and thereafter, and therefore, it was suggested that the expression thereof was regulated differently from the expression of PD-1. In B4-deficient CD8-positive T cells, the effect on the expression of PD-1 by activation stimulation with the anti-CD3 antibody/the anti-CD28 antibody was not observed, but attenuation of the expression of Tim-3 was observed.

In FIG. 3, the expression of B4, PD-1, and Tim-3 in mouse naive CD4-positive T cells is shown. As shown in FIG. 3, in the mouse naive CD4-positive T cells, a weak but apparent expression of PD-1 was observed, but the expression of B4 and Tim-3 was not observed. In addition, the effect on the expression of PD-1 or Tim-3 by B4 deletion was not observed.

In FIG. 4, the expression of B4, PD-1, and Tim-3 after stimulation with the anti-CD3 antibody/the anti-CD28 antibody in mouse CD4-positive T cells is shown. As shown in FIG. 4, by activation stimulation with the anti-CD3 antibody/the anti-CD28 antibody, the expression of PD-1 on the mouse CD4-positive T cells was almost maximized on day 1, but the expression of B4 and Tim-3 slightly increased on day 1, and was maximized on day 2 and thereafter, and therefore, it was suggested that the expression thereof was regulated differently from the expression of PD-1. In B4-deficient CD4-positive T cells, the effect on the expression of PD-1 by activation stimulation with the anti-CD3 antibody/the anti-CD28 antibody was not observed, but attenuation of the expression of Tim-3 was observed.

In FIG. 5, the expression of B4 and PD-1 after stimulation with the anti-CD3 antibody/the anti-CD28 antibody in mouse CD8-positive T cells and CD4-positive T cells is shown. As shown in FIG. 5, in both the mouse CD8-positive T cells and CD4-positive T cells, the expression of PD-1 first increased, and thereafter, the expression of B4 increased late, and therefore, it was found that both T cells became PD-1 and B4 double positive-cells.

From the above results, it was revealed that B4 was expressed in mouse T cells, and was an immune checkpoint molecule different from PD-1 and Tim-3.

[Example 2] Expression of LILRB4 in Human T Cells

To a 48-well plate (manufactured by Thermo Fisher Scientific, Inc., #150687), 100 μL of a PBS(-) buffer solution containing each of an anti-human CD3 antibody (manufactured by BD Biosciences, Inc., Clone: UCHT1, #555329) and an anti-human CD28 antibody (manufactured by BD Biosciences, Inc., Clone: CD28.2, #555725) at a concentration of 10 pg/mL was added to coat the wells therewith at room temperature for 30 minutes, followed by washing the wells three times with 500 μL of a PBS(-) buffer solution. Frozen human peripheral blood mononuclear cells (manufactured by C.T.L.) were rapidly thawed in a warm bath at 37°C, and suspended in RPMI-1640 medium (manufactured by Sigma-Aldrich Co. LLC, #R8758) containing 10% fetal bovine serum, 50 μM 2-mercaptoethanol, a 1% penicillin (5000 U/mL)/streptomycin (5000 μg/mL) solution, and 20 U/mL DNase I (manufactured by Sigma-Aldrich Co. LLC, #D5025) and cultured overnight, and thereafter, the cells were recovered. The T cells were purified using Naive Pan T cell Isolation Kit, human (manufactured by Miltenyi Biotec K.K., #130-097-095) and suspended in a DNase I-free medium having the above composition. In the wells coated with the antibody, the suspended T cells were seeded at a concentration of $1 \times 10^6$ cells/200 μL/well and cultured at 37°C under 5% carbon dioxide conditions for 0 to 3 days. The cells were recovered from the wells, and thereafter suspended in a PBS(-) buffer solution containing 2% fetal bovine serum and 0.05% sodium azide and stained under ice temperature conditions using an FITC-labeled anti-human CD4 antibody (manufactured by BioLegend, Clone: RPA-T4, #300506), an Alexa 647-labeled anti-human CD8a antibody (manufactured by BioLegend, Clone: RPA-T8, #301022), a PE-labeled anti-human CD85k (LILRB4) antibody (manufactured by eBioscience, Inc., Clone: ZM4.1, #12-5139-42), a BV421-labeled anti-human PD-1 antibody (manufactured by BioLegend, Clone: EH12.2H7, #329920), or PerCP-Cy5.5-labeled anti-human Tim-3 antibody (manufactured by BioLegend, Clone: F38-2E2, #345016), or as an isotype control antibody, PE-labeled mouse IgG1, k (manufactured by eBioscience, Inc., Clone: P3.6.2.8.1, #12-4714-42), BV421-labeled mouse IgG1 (manufactured by BioLegend, Clone: MOPC-21, #400158), or PerCP-Cy5.5-labeled mouse IgG1 (manufactured by BD Biosciences, Inc., Clone: MOPC-21, #552834), and measured by BD FACS Aria III cell sorter (manufactured by BD Biosciences, Inc.), and data were analyzed by FlowJo software (manufactured by BD Biosciences, Inc.) .

In FIG. 6, the expression of B4, PD-1, and Tim-3 after stimulation with the anti-CD3 antibody/the anti-CD28 antibody in human CD8-positive T cells and CD4-positive T cells is shown. As shown in FIG. 6, in the human CD8-positive T cells and CD4-positive T cells, the expression of any of B4, PD-1, and Tim-3 was not observed with no stimulation (on day 0). However, an increase in the expression of B4 was observed by activation stimulation with the anti-CD3 antibody/the anti-CD28 antibody in the same manner as that of PD-1 and Tim-3.

From the above results, it was revealed that B4 was expressed in human T cells, and was an immune checkpoint molecule different from PD-1 and Tim-3.

[Example 3] Analysis of Binding Site of Fibronectin to LILRB4

Human fibronectin is translated from various mRNA isoforms transcribed from a single gene located at 2q35, and is generally composed of 2240 to 2483 amino acid residues including a signal peptide of 26 residues. Fibronectin exists as a soluble dimer in plasma, and exists as a dimer or a multimer on the cell surface or the extracellular matrix (ECM). The dimeric fibronectin has a structure in which two substantially identical polypeptides of 210 kDa to 250 kDa are bound through two disulfide bonds near the C terminus, and each polypeptide is constituted by many functional modules, and these modules have a property of binding to other proteins including fibrin, collagen, an integrin, a heparin, a syndecan, and fibronectin.

In order to identify a B4 binding site in fibronectin, B4 binding affinity was analyzed by subjecting individual domains including these modules to a reporter cell assay and a BLI (Bio-layer interferometry) analysis.

The BLI analysis was performed as follows using BLItz. Human His-tagged human LILRB4 was immobilized on a Ni-NTA sensor, and unbound proteins were washed off with phosphate buffered saline (PBS). The sensor was immersed in a test protein and then immersed in PBS for dissociation. Curve regression and data processing were performed with BLItz Pro software.

The reporter cell assay was performed by transfecting a mouse T cell hybridoma cell line that expresses NFAT-GFP reporter gene and DAP12 with a retrovirus vector using a chimeric receptor in which the extracellular domain is human LILRB4, and the transmembrane domain and the intracellular domain are each an activated paired immunoglobulin-like receptor beta, culturing the obtained $5 \times 10^4$ reporter cells with a test protein, and analyzing the expression of GFP by flow cytometry.

As a result, an N-terminal cathepsin D digested fragment 70 kDa polypeptide of human fibronectin and a 30 kDa fragment at the N-terminal side that is a trypsin digested fragment thereof had binding affinity, but a 45 kDa fragment at the C-terminal side did not have binding affinity in the reporter cell assay and the BLI analysis. A moiety at a side closer to the C terminus of the fibronectin, that is, the amino acid residues 607 to 1265, 1266 to 1908, and 1277 to 2477 did not induce a signal even in the reporter cell assay or in the BLI analysis. From the results, it was indicated that the B4 binding site in the fibronectin is present in the N-terminal 30 kDa fragment (FN30).

Further, when peptide mapping was performed by overlapping 8 residues at every 20 amino acid residues of FN30, only an amino acid sequence from Cys123 to His142 (CysThrCysIleGlyAlaGlyArgGlyArgIleSerCysThrIleAlaAsnArgCys His) induced a prominent signal in the reporter cells. The amino acid sequence from Cys123 to His142 of the fibronectin is represented by SEQ ID NO: 1. From the results, it was revealed that the fibronectin bound to B4 via the amino acid sequence represented by SEQ ID NO: 1.

[Example 4] Preparation of Recombinant Fibronectin Target Sequence-Fc Fusion Protein

A recombinant protein (hereinafter also referred to as FN30-Fc) in which the N-terminal 30 kDa of human fibronectin (corresponding to a glutamine residue at position 40 to a glycine residue at position 282, manufactured by Sigma-Aldrich Co. LLC, #9911, hereinafter also referred to as FN30) was fused with Fc of mouse IgG2a was prepared by the following procedure.

A DNA fragment encoding FN30 was amplified from mRNA derived from human mesenchymal stem cells using the following primers.

FN30 Forward Primer: 5'-ATAGAATTCGCAGTCCCCGGTGGCTGTCAGT-3'(SEQ ID NO: 2)
FN30 Reverse Primer: 5'-TTAAGATCTTCCGCTCGATGTGGTCTGCAC-3'(SEQ ID NO: 3)

The amplified FN30 DNA fragment was digested with EcoRI and BglII and inserted into the respective sites of a pFUSE-mIgG2Ae1-Fc2 vector. This vector is a vector resulting from introduction of mutations of L235E, E318A, K320A, and K322A into the Fc moiety so as to reduce the antibody-dependent cellular cytotoxicity (ADCC) and complement-dependent cytotoxicity (CDC) inherent in Fc of IgG2a.

The obtained plasmid pFUSE-mIgG2Ae1-Fc2/FN30 was confirmed by performing a sequence analysis, and then used for protein expression. For expression of FN30-Fc, this plasmid was transfected into CHO-K1 cells using Lipofectamine 2000, and stably transfected cell clones were selected using Zeocin (0.8 mg/mL), and then obtained by a limiting dilution method. Recombinant FN30-Fc was purified from the cell culture supernatant with a HiTrap Protein G HP column and then dialyzed against PBS(-).

[Example 5] Preparation of Anti-Fibronectin Antibody

A WKY rat was immunized with a 30 kDa fragment at an N-terminal side (FN30; manufactured by Sigma-Aldrich Co. LLC, #9911) of fibronectin, which was obtained by treating human plasma-derived fibronectin with cathepsin D, and then treating the resulting 70 kDa fragment with trypsin, followed by purification by utilizing heparin binding affinity, as an antigen, and iliac lymph node cells were fused with myeloma cells in the presence of 50% polyethylene glycol, whereby hybridomas were obtained. By performing selection by ELISA using human FN30, FN30-Fc obtained in Example 4, mouse FN (manufactured by Abcam. plc, #ab92784), and a synthetic peptide fragment of FN30, 9 clones of hybridomas that produce an anti-fibronectin monoclonal antibody (hereinafter also referred to as "anti-FN30 monoclonal antibody") were obtained. The clone name, isotype, binding affinity for mouse MSC, and binding affinity for human MSC of the established 9 types of anti-FN30 monoclonal antibodies are shown in Table 1. In the table, (-) indicates no binding affinity, (+) indicates weak binding affinity, and (++) indicates strong binding affinity.

[Table 1]

| Antibody number | Isotype (rat IgG) | Binding affinity for mouse MSC | Binding affinity for human MSC |
|---|---|---|---|
| 1 | IgG2b | - | - |
| 2 | IgG2a | - | |
| 3 | IgG2b | - | - |
| 4 | IgG2a | ++ | ++ |
| 5 | IgG2a | ++ | ++ |
| 6 | IgG2b | ++ | ++ |
| 7 | IgG2c | + | + |
| 8 | IgG2a | - | - |
| 9 | IgG2a | - | + |

[Example 6] Blocking Effect of Anti-Fibronectin Antibody

Human bone marrow-derived mesenchymal stem cells (manufactured by PromoCell GmbH, #C-12974) were suspended in a mesenchymal stem cell growth medium (manufactured by PromoCell GmbH, C-28009) and seeded into a 48-well plate at $1 \times 10^5$ cells/300 μL/well and cultured at 37°C under 5% carbon dioxide conditions. The medium was removed by suction in a state where the mesenchymal stem cells sufficiently adhered to the plate after 24-hour culturing, and 300 μL of a PBS(-) buffer solution containing each of the anti-FN30 monoclonal antibodies (No. 1 to No. 9) obtained in Example 5 at 20 μg/mL was added thereto, and a reaction was allowed to proceed at 37°C for 1 hour. Thereafter, the PBS(-) buffer solution containing the anti-FN30 monoclonal antibody was removed by suction, the wells were washed

twice with 500 μL of RPMI-1640 medium containing 0.5% fetal bovine serum, 50 μM 2-mercaptoethanol, and a 1% penicillin (5000 U/mL)/streptomycin (5000 μg/mL) solution, and B4 chimeric receptor GFP reporter cells (B4-2B4 cells) or control GFP reporter cells (2B4 cells) expressing no chimeric receptor suspended in RPMI-1640 medium with the same composition were seeded at $1\times10^5$ cells/200 μL/well and cultured at 37°C under 5% carbon dioxide conditions for 18 hours. After recovering the cells from the wells, the cells were suspended in a PBS(-) buffer solution containing 2% fetal bovine serum and 0.05% sodium azide, and measured by BD FACS Calibur flow cytometer (manufactured by BD Biosciences, Inc.), and data were analyzed by FlowJo software. The results are shown in FIG. 7.

As shown in FIG. 7, when the treatment was performed with the anti-FN30 monoclonal antibodies Nos. 1 to 5 and 7 to 9, GFP expression equivalent to that of the positive control without antibody was shown, however, when the treatment was performed with the anti-FN30 monoclonal antibody No. 6, the GFP expression was equivalent to that of the negative control of only the reporter cells, and therefore, it can be said that the antibody No. 6 is an antibody that inhibits binding between FN30 and B4.

[Example 7] Cross-Reactivity of Each Anti-Human FN30 Monoclonal Antibody (Staining of Mouse Bone Marrow-Derived Mesenchymal Stem Cells (MSC) and Human Bone Marrow-Derived Mesenchymal Stem Cells)

Mouse bone marrow-derived mesenchymal stem cells (manufactured by Cyagen Biosciences, Inc., #MUBMX-01001) and human bone marrow-derived mesenchymal stem cells recovered from plates by pipetting without performing an enzyme treatment or the like were suspended in a PBS(-) buffer solution containing 2% fetal bovine serum and 0.05% sodium azide, treated with each of the anti-FN30 monoclonal antibodies obtained in Example 5 or PE-labeled rat IgG1, k (manufactured by BD Biosciences, Inc., Clone: R3-34, #553925) under ice temperature conditions, washed, and then stained with a PE-labeled goat anti-rat IgG polyclonal antibody (manufactured by BioLegend, #405406), and measured by BD FACS Calibur flow cytometer, and data were analyzed by FlowJo software. The results are shown in FIG. 8.

As shown in FIG. 8, the anti-FN30 monoclonal antibodies Nos. 4, 5, and 6 strongly stained both mouse MSC and human MSC, and the antibody No. 7 weakly stained mouse MSC and human MSC. The antibody No. 9 weakly stained only human MSC. The antibodies Nos. 1, 2, 3, and 8 did not stain either MSC.

[Example 8] Cell Surface Expression of Fibronectin (FN30) in Human and Mouse Cancer Cell Lines

Mouse cancer cell lines: B16F10 (melanoma) and 3LL (Lewis lung cancer), and human cancer cell lines: Daudi (Burkitt lymphoma), HL60 (promyelocytic leukemia), HeLa (cervical epithelioid carcinoma), HepG2 (hepatocellular carcinoma), and Saos-2 (osteosarcoma), each recovered from plates by pipetting without performing an enzyme treatment or the like, were suspended in a PBS(-) buffer solution containing 2% fetal bovine serum and 0.05% sodium azide, treated with the anti-FN30 monoclonal antibody (No. 5) or PE-labeled rat IgG2a, k (manufactured by BD Biosciences, Inc., Clone: R35-95, #554689) under ice temperature conditions, washed, and then stained with an Alexa 488-labeled goat anti-rat IgG polyclonal antibody (manufactured by Invitrogen, Inc., #A-11006), and measured by BD FACS Calibur flow cytometer, and data were analyzed by FlowJo software. The results are shown in FIG. 9.

As shown in FIG. 9, it was found that the expression of fibronectin (FN30) on the cell surface in the mouse cancer cell lines was weak in B16F10 and strong in 3LL. In the human cancer cell lines, the cell surface expression was not observed in Daudi, HL60, and HeLa, and strong expression on the cell surface was observed in HepG2 and Saos-2.

[Example 9] Expression of B4 and PD-1 in Intratumoral Infiltrating Lymphocytes

Into the left thigh of a wild-type mouse, B16F10 cells or 3LL cells were subcutaneously inoculated at $5\times10^5$ cells/100 pL/mouse, and thereafter, the mouse was euthanized by excessive inhalation of carbon dioxide at a stage when the diameter grew to about 1.5 cm. Then, the tumor was taken out and a single cell suspension was prepared using Tumor Dissociation Kit, mouse (manufactured by Miltenyi Biotec K.K., #130-096-730) and gentleMACS Dissociator (manufactured by Miltenyi Biotec K.K., #130-093-235), and lymphocytes were recovered by specific gravity separation using Percoll (manufactured by GE Healthcare, Inc., #17089102). The recovered cells were suspended in a PBS(-) buffer solution containing 2% fetal bovine serum and 0.05% sodium azide, and stained under ice temperature conditions with an FITC-labeled anti-mouse CD4 antibody, an Alexa 647-labeled anti-mouse CD8a antibody, a PE-labeled anti-mouse gp49A/B antibody, or a BV421-labeled anti-mouse PD-1 antibody, or as an isotype control antibody, PE-labeled Armenian hamster IgG, or BV421-labeled rat IgG2a, and measured by BD FACS Aria III cell sorter, and data were analyzed by FlowJo software. The results are shown in FIGS. 10 and 11.

In FIG. 10, the expression of B4 and PD-1 in intratumoral infiltrating lymphocytes (mouse CD8-positive T cells) is shown. As shown in FIG. 10, the expression of B4 and PD-1 was observed in some of the CD8-positive T cells infiltrating into the tumor of B16F10, and most of them were B4 and PD-1 double positive-cells, and also B4 single positive-cells were observed. Most of the CD8-positive T cells infiltrating into the tumor of 3LL were positive for B4, and 80% or more of them were also positive for PD-1.

In FIG. 11, the expression of B4 and PD-1 of intratumoral infiltrating lymphocytes (mouse CD4-positive T cells) is shown. As shown in FIG. 11, the expression of B4 and PD-1 was observed in some of the CD4-positive T cells infiltrating into the tumor of B16F10, and B4 and PD-1 double-positive cells and B4 single-positive cells were also observed. Most of the CD4-positive T cells infiltrating into the tumor of 3LL were B4 and PD-1 double-positive cells.

[Example 10] Protein Detection in Plasma Sample Using Anti-Fibronectin Polyclonal Antibody and Anti-Fibronectin Monoclonal Antibody (Western Blot Method)

As a plasma sample, blood of a healthy male (age: 25 to 31 years old) was used, and 10.5 mL/person of blood was collected in a Venoject II vacuum blood collection tube (registered trademark), and plasma was separated and recovered by centrifugation at 2500 rpm for 3 minutes using a universal cooling centrifuge (KUBOTA S911).

The plasma sample was diluted 15-fold with ultrapure water, and a reducing agent (1 M $\beta$-ME) and a surfactant (4% SDS) were added thereto, and the resulting mixture was heated at 95°C for 5 minutes, and then electrophoresed in a 7.5% acrylamide gel under reducing conditions. After electrophoresis, the separated protein was transferred to a polyvinylidene fluoride (PVDF) membrane. The obtained blots were stained using Pierce ECL Western Blotting Substrate manufactured by Thermo Fisher Scientific, Inc. as a substrate after adding each of an anti-FN polyclonal antibody and the anti-FN30 monoclonal antibodies Nos. 4 and 5 obtained in Example 5 as a primary antibody, and each of an anti-rabbit IgG-HRP antibody (manufactured by Cell Signaling Technology, Inc., #7074) and an anti-rat IgG-HRP antibody (manufactured by BioLegend, Clone: Poly4054, #405405) as a secondary antibody. In an image analysis, ImageQuant LAS 4000 mini manufactured by GE Healthcare, Inc. was used. The results are shown in FIG. 12.

As shown in FIG. 12, a band of 250 kDa was confirmed when using the anti-FN30 monoclonal antibodies Nos. 4 and 5. A band having a molecular weight of about 24 kDa was observed when using either of the anti-FN polyclonal antibody and the anti-FN30 monoclonal antibodies, and the existence of an FN fragment of 24 kDa in the healthy human plasma was confirmed.

[Example 11] Quantitative Determination of Full-Length Molecule of Fibronectin and 24 kDa Fragment of Fibronectin in Healthy Human Plasma Using Anti-Fibronectin Antibody

(ELISA Method)

The anti-FN30 monoclonal antibody No. 6 obtained in Example 5, an anti-FN44 kDa antibody (manufactured by OriGene Technologies, Inc., Clone: OTI3F9, hereinafter also referred to as "anti-FN44 antibody") using Collagen binding-FN as an antigen, and Anti-Fibronectin antibody produced in rabbit (manufactured by Sigma-Aldrich Co. LLC, #F3648, hereinafter also referred to as "anti-FN polyclonal antibody") that is a polyclonal antibody were used, and in a 96-well plate (manufactured by Greiner Bio-One GmbH, MICROLON (registered trademark)), each monoclonal antibody was diluted to a final concentration of 4 $\mu$g/mL with a carbonate buffer (NaHCO$_3$ 0.01 M), and the plate was coated therewith by being left to stand at 4°C for 12 hours. After the plate was blocked with PBS containing 1% BSA, standard FN protein (manufactured by R & D systems, Inc., Fibronectin ELISA DuoSet) and healthy human plasma were added thereto.

The amount of fibronectin bound to the monoclonal antibody was detected using an anti-FN polyclonal antibody (13500-fold dilution) and an anti-rabbit IgG-HRP antibody (250-fold dilution). In plate washing between each operation, TBS with 0.1% Tween 20 was used. After adding the substrate, the absorbance was measured at a wavelength of 450 nm with a microplate reader (manufactured by Bio-Rad Laboratories, Inc., Model 680) and digitized.

Nonlinear approximation was performed by 4-parameter logistic regression based on the absorbance of the standard protein, and the concentration was calculated from the absorbance value of the sample. The results are shown in FIG. 13. The fibronectin concentration [A ($\mu$g/mL)] detected using the anti-FN30 monoclonal antibody, and the fibronectin concentration [B ($\mu$g/mL)] detected using the anti-FN44 antibody are considered to be the concentrations shown in FIG. 13.

When the full-length molecule of fibronectin is represented by x $\mu$g/mL, 24 kDa-deficient fibronectin is represented by y $\mu$g/mL, and 24 kDa fibronectin is represented by z $\mu$g/mL, these satisfy a relationship of the following formulae when considering that the 24 kDa-deficient fibronectin and the 24 kDa fibronectin are both degradation products and the polyclonal antibody to which the secondary antibody binds is approximately proportional to the molecular weight.

[Math. 1]

$$A = x + y, \quad B = x + z, \quad y = \frac{227}{24} z$$

From the formulae, x, y, and z are calculated according to the following formulae.

[Math. 2]

$$x = \frac{227}{203} A - \frac{24}{203} B, \quad y = \frac{227}{203} (B - A), \quad z = \frac{24}{203} (B - A)$$

The results are shown as boxplot displays representing the first, second, and third quartiles, and the maximum value, and the minimum value (FIG. 14). As a result, the concentration of the full-length molecule of fibronectin was 279 ± 131 µg/mL, which was in agreement with the known FN concentration (300 µg/mL). By utilizing the difference of each concentration using the anti-FN30 monoclonal antibody No. 6 and the anti-FN44 antibody, the FN24 kDa concentration in the plasma was calculated to be 6.49 ± 7.44 µg/mL.

[Example 12] Therapeutic Effect of FN30 on Autoantibody Disease

It was verified whether or not inhibition of FN30 inhibits binding between gp49B and fibronectin and exhibits a therapeutic effect on the production of an autoantibody by pathogenic plasma cells as follows.
Intraperitoneal administration of a control IgG or FN30-Fc obtained in Example 4 to BXSB/Yaa mice was repeated twice at an interval of 2 weeks. The results are shown in FIG. 15.
As shown in FIG. 15, in a group of mice to which the control IgG was administered, the anti-dsDNA IgG serum antibody titer gradually increased, however, in the FN30-Fc administration group, the IgG autoantibody level was kept relatively constant during the observed period.
This inhibitory effect was also similarly observed by intraperitoneally administering the anti-gp49B monoclonal antibody H1.1 to BXSB/Yaa mice on the same schedule as that for the administration of FN30-Fc (FIG. 16). Suppression of the increase in autoantibody titer did not have a particular effect on the spleen weight, the total spleen cell count, the total bone marrow cell count, the ratio of plasma cells in the spleen and in the bone marrow, the frequency of occurrence of dsDNA autoantibody-producing cells in the spleen, and the like.
These results suggested that the administration of FN30-Fc or the anti-gp49B monoclonal antibody H1.1 to BXSB/Yaa mice suppressed a further increase in anti-dsDNA IgG without particularly eliminating the antibody-producing cells. From these results, it is considered that FN-30 and the anti-B4 antibody have an effect of alleviating an autoimmune disease by blocking binding between B4 and FN.

[Example 13] Involvement of LILRB4 in Cancer Metastasis

In order to evaluate whether or not LILRB4 (gp49B in mice) is involved in tumor metastasis, Lewis lung carcinoma cells (LLC) or mouse melanoma cells B16F10 were injected into wild-type (WT) B6 mice and gp49B-deficient mice through the tail vein, and after 30 days and 20 days, respectively, the lung and the liver were excised, and H & E staining was performed or the number of tumor nodules on the surface was counted and evaluation was performed. The results of LLC injection are shown in FIGS. 17A to 17D, and the results of B16F10 injection are shown in FIGS. 18A, 18B and 19.
As shown in FIGS. 17A and 17B, the metastasis of LLC to the lung was reduced in the gp49B-deficient mice as compared to WT. In addition, as shown in FIGS. 17C and 17D, the site of tumor metastasis was found only in the liver of the WT mice injected with LLC, and was not observed in the liver of the gp49B-deficient mice.
With respect to B16F10, as shown in FIGS. 18A and 18B, the number of tumor nodules on the total surface of the lung was dominantly reduced in the gp49B-deficient mice (FIG. 18A), and metastasis to the liver was also reduced (FIG. 18B).
Subsequently, the whole body radiation of C57BL/6NJcl mice was performed at 8.5 Gy, and after 1 day, bone marrow cells derived from the wild-type or gp49B-deficient mouse were injected and transferred to the mice through the tail vein at $5 \times 10^6$ cells/mouse. After adoptive transfer, the mice were treated with gentamycin for 1 month, and at 4 weeks after the transfer, the total blood image was confirmed for the replacement state of donor cells by a flow cytometer.

The results are shown in FIG. 19. As shown in FIG. 19, metastasis to the lung and the liver was lower in the mouse in which the bone marrow cells of the gp49B-deficient mouse were adoptively transferred than in the control mouse in which the bone marrow cells of the wild-type mouse were adoptively transferred. From these results, it was demonstrated that gp49B is involved in tumor cell metastasis.

[Example 14] Cancer Metastasis Inhibitory Effect by Inhibition of LILRB4

As shown in Example 12, tumor metastasis is reduced when gp49B is deficient, and therefore, it was examined how tumor metastasis is affected by inhibition of gp49B using the anti-gp49B monoclonal antibody H1.1 as follows.

B6 mice injected with B16F10 were intraperitoneally injected 6 times with a control isotype IgG antibody, an anti-PD-1 monoclonal antibody, an anti-gp49B monoclonal antibody, or a combination of an anti-PD-1 monoclonal antibody and an anti-gp49B monoclonal antibody. After administration of the antibody, the number of tumor nodules in the lung and the number of metastatic foci in the liver were evaluated. The results are shown in FIGS. 20A to 20D.

As shown in FIGS. 20A to 20D, in the anti-gp49B monoclonal antibody-treated group, the number of metastatic foci of B16F10 tumor in both the lung and the liver decreased in the same manner as in the anti-PD-1 monoclonal antibody-treated group, or in the group treated with the anti-gp49B monoclonal antibody and the anti-PD-1 monoclonal antibody in combination. Further, when LLC expressing luciferase (LLC-Luc2) was injected into B6 mice and the effect of the control isotype IgG antibody, the anti-PD-1 monoclonal antibody, the anti-gp49B monoclonal antibody, or a combination of the anti-PD-1 monoclonal antibody and the anti-gp49B monoclonal antibody was examined, in the anti-gp49B monoclonal antibody-treated group, and in the group treated with the anti-PD-1 monoclonal antibody and the anti-gp49B monoclonal antibody in combination, the number of metastatic foci of LLC-Luc2 tumor decreased in both the lung and the liver (FIGS. 20E to 20G).

Further, when the anti-PD-1 monoclonal antibody and the anti-gp49B monoclonal antibody were used in combination in mice having an LLC-Luc2 tumor, the number of metastatic foci was statistically significantly decreased (FIG. 20G). The inhibition of gp49B suppressed metastasis of LLC-Luc2 in the lung and the liver also in an analysis of a pathological section in the same manner as the simultaneous inhibition of PD-1 and gp49B (FIG. 21). From these results, it was revealed that the inhibition of LILRB4 suppresses cancer metastasis.

[Example 15] Verification 1 of Effect of Promoting Osteoclast Differentiation by Inhibition of LILRB4

Bone marrow cells were collected from a wild-type B6 mouse and cultured in a 48-well plate at $5.0 \times 10^5$ cells/well using $\alpha$-MEM medium containing 10% FCS and 10 $\mu$g/mL of a control isotype IgG antibody, the anti-gp49B monoclonal antibody H1.1, a $F(ab')_2$ fragment of the anti-gp49B monoclonal antibody H1.1, or the anti-FN30 monoclonal antibody No. 6. After 2-hour culturing, M-CSF was added at 20 ng/well, and culturing was further performed for 48 hours. From this point of time, in order to induce differentiation into osteoclasts, $\alpha$-MEM supplemented with 10% FCS containing 20 ng/mL M-CSF and 100 ng/mL RANKL was further added thereto, and after 6 days, TRAP staining was performed. The results are shown in FIG. 22.

As shown in FIG. 22, as for the induction of differentiation into osteoclasts, by the addition of the anti-gp49B monoclonal antibody H1.1 or the $F(ab')_2$ fragment of the anti-gp49B monoclonal antibody H1.1, the induction differentiation rate (%) (number of osteoclasts/total number of cells $\times$ 100) was 3.5% and 3.4%, respectively, and a slightly increasing trend was observed as compared to the case of the control isotype IgG antibody (2.7%). Further, the induction differentiation rate was 7.9% by the addition of the anti-FN30 monoclonal antibody No. 6, and a significant increase was observed.

[Example 16] Verification 2 of Effect of Promoting Osteoclast Differentiation by Inhibition of LILRB4

CD11b-positive monocytes were obtained from peripheral blood mononuclear cells (PBMC) prepared from a blood sample collected from a healthy subject using a magnetic cell sorter MACS (manufactured by Miltenyi Biotec K.K.). The monocytes were cultured for 7 days in $\alpha$-MED medium supplemented with 10% FCS containing 100 ng/mL RANKL and 25 ng/mL M-CSF by adding an anti-LILRB4 monoclonal antibody ZM4.1 (manufactured by Thermo Fisher Scientific, Inc.) at 1.0 $\mu$g/mL or Mouse IgG1$\kappa$ (manufactured by BioLegend) at 1.0 $\mu$g/mL as a control isotype antibody, followed by TRAP staining. The results are shown in FIG. 23.

As shown in FIG. 23, the induction differentiation rate from PBMC into osteoclasts was 13.7%, and as compared to the case of the control isotype antibody (7.6%), the induction of differentiation into osteoclasts was increased to about twice by the addition of the anti-LILRB4 antibody.

[Example 17] Verification 3 of Effect of Promoting Osteoclast Differentiation by Inhibition of LILRB4

Bone marrow cells were collected from a wild-type B6 mouse and a gp49B-deficient mouse and cultured in a 24-well plate for 2 days using $\alpha$-MEM medium containing 10% FCS, 20 ng/mL M-CSF, and $1.0 \times 10^6$ cells/mL. Thereafter, in order to induce differentiation into osteoclasts, $\alpha$-MEM supplemented with 10% FCS containing M-CSF at 20 ng/well and RANKL at 100 ng/mL was added thereto, and after 5 days, TRAP staining was performed. The results are shown in FIG. 24.

As shown in FIG. 24, the induction differentiation rate into osteoclasts increased to approximately 3 times in the bone marrow cells derived from the gp49B-deficient mouse as compared to the bone marrow cells derived from the wild-type mouse.

[Example 18] Verification 4 of Effect of Promoting Osteoclast Differentiation by Inhibition of LILRB4

The femurs were isolated from a wild-type B6 mouse (female) at 18 weeks of age and a gp49B-deficient mouse (female) at 18 weeks of age, and embedded according to the Kawamoto's method ("Technique for Preparation of Thin Frozen Sections from Undecalcified Hard Tissues (Kawamoto's method 2008) and its Application", Tadafumi Kawamoto, "Pathological Technology, vol. 72, No. 2, pp. 76-83, 2009"), and thereafter, a frozen section was prepared, followed by TRAP staining, whereby osteoclasts were detected. The results are shown in FIG. 25.

As shown in FIG. 25, more osteoclasts were detected in the gp49B-deficient mouse than in the wild-type B6 mouse.

Industrial Applicability

According to the present invention, an immune checkpoint inhibitor, a therapeutic agent for an immune checkpoint-related disease, an immunosuppressant, an anti-fibronectin antibody or a derivative thereof, a fibronectin analog, a kit for detecting fibronectin or a partial protein thereof, and a method for detecting fibronectin or a partial protein thereof can be provided.

**Claims**

1. An immune checkpoint inhibitor, comprising a substance that inhibits binding between fibronectin and an immunosuppressive receptor LILRB4 as an active ingredient.

2. The immune checkpoint inhibitor according to Claim 1, wherein the fibronectin binds to the immunosuppressive receptor LILRB4 via the amino acid sequence represented by SEQ ID NO: 1 in the fibronectin.

3. The immune checkpoint inhibitor according to Claim 1 or 2, wherein the substance that inhibits binding between the fibronectin and the immunosuppressive receptor LILRB4 is an anti-fibronectin antibody or a derivative thereof, an anti-immunosuppressive receptor LILRB4 antibody or a derivative thereof, or a fibronectin analog.

4. The immune checkpoint inhibitor according to Claim 3, wherein the anti-fibronectin antibody or a derivative thereof binds to the amino acid sequence represented by SEQ ID NO: 1 in the fibronectin.

5. The immune checkpoint inhibitor according to Claim 3, wherein the fibronectin analog is any one of the following peptides (a) to (c):

   (a) a peptide containing the amino acid sequence represented by SEQ ID NO: 1;
   (b) a peptide containing an amino acid sequence in which one to several amino acids have been deleted, inserted, substituted, or added in the amino acid sequence represented by SEQ ID NO: 1, and having a binding capacity to a fibronectin binding site of the immunosuppressive receptor LILRB4; and
   (c) a peptide containing an amino acid sequence having 80% or more identity to the amino acid sequence represented by SEQ ID NO: 1, and having a binding capacity to a fibronectin binding site of the immunosuppressive receptor LILRB4.

6. A therapeutic agent for an immune checkpoint-related disease, comprising a substance that inhibits binding between fibronectin and an immunosuppressive receptor LILRB4 as an active ingredient.

7. The therapeutic agent according to Claim 6, wherein the fibronectin binds to the immunosuppressive receptor LILRB4 via the amino acid sequence represented by SEQ ID NO: 1 in the fibronectin.

8. The therapeutic agent according to Claim 6 or 7, wherein the substance that inhibits binding between the fibronectin and the immunosuppressive receptor LILRB4 is an anti-fibronectin antibody or a derivative thereof, an anti-immunosuppressive receptor LILRB4 antibody or a derivative thereof, or a fibronectin analog.

9. The therapeutic agent according to Claim 8, wherein the anti-fibronectin antibody or a derivative thereof binds to the amino acid sequence represented by SEQ ID NO: 1 in the fibronectin.

10. The therapeutic agent according to Claim 8, wherein the fibronectin analog is any one of the following peptides (a) to (c):

   (a) a peptide containing the amino acid sequence represented by SEQ ID NO: 1;
   (b) a peptide containing an amino acid sequence in which one to several amino acids have been deleted, inserted, substituted, or added in the amino acid sequence represented by SEQ ID NO: 1, and having a binding capacity to a fibronectin binding site of the immunosuppressive receptor LILRB4; and
   (c) a peptide containing an amino acid sequence having 80% or more identity to the amino acid sequence represented by SEQ ID NO: 1, and having a binding capacity to a fibronectin binding site of the immunosuppressive receptor LILRB4.

11. The therapeutic agent according to any one of Claims 6 to 10, wherein the immune checkpoint-related disease is selected from the group consisting of an autoimmune disease, a cancer, an inflammatory disease, and an allergic disease.

12. The therapeutic agent according to Claim 11, wherein the cancer is due to cancer metastasis.

13. A therapeutic agent for an immune checkpoint-related disease, comprising a substance that activates an immunosuppressive receptor LILRB4 as an active ingredient.

14. The therapeutic agent according to Claim 13, wherein the substance that activates the immunosuppressive receptor LILRB4 is an anti-fibronectin antibody or a derivative thereof, an anti-immunosuppressive receptor LILRB4 antibody or a derivative thereof, or fibronectin or a fibronectin analog.

15. The therapeutic agent according to Claim 14, wherein the fibronectin analog is any one of the following peptides (a) to (c):

(a) a peptide containing the amino acid sequence represented by SEQ ID NO: 1;
(b) a peptide containing an amino acid sequence in which one to several amino acids have been deleted, inserted, substituted, or added in the amino acid sequence represented by SEQ ID NO: 1, and having a binding capacity to a fibronectin binding site of the immunosuppressive receptor LILRB4; and
(c) a peptide containing an amino acid sequence having 80% or more identity to the amino acid sequence represented by SEQ ID NO: 1, and having a binding capacity to a fibronectin binding site of the immunosuppressive receptor LILRB4.

16. The therapeutic agent according to any one of Claims 13 to 15, wherein the immune checkpoint-related disease is a bone disease.

17. An immunosuppressant, comprising a substance that activates an immunosuppressive receptor LILRB4 as an active ingredient.

18. The immunosuppressant according to Claim 17, wherein the substance that activates the immunosuppressive receptor LILRB4 is an anti-fibronectin antibody or a derivative thereof, an anti-immunosuppressive receptor LILRB4 antibody or a derivative thereof, or fibronectin or a fibronectin analog.

19. The immunosuppressant according to Claim 18, wherein the fibronectin analog is any one of the following peptides (a) to (c):

(a) a peptide containing the amino acid sequence represented by SEQ ID NO: 1;
(b) a peptide containing an amino acid sequence in which one to several amino acids have been deleted, inserted, substituted, or added in the amino acid sequence represented by SEQ ID NO: 1, and having a binding capacity to a fibronectin binding site of the immunosuppressive receptor LILRB4; and
(c) a peptide containing an amino acid sequence having 80% or more identity to the amino acid sequence represented by SEQ ID NO: 1, and having a binding capacity to a fibronectin binding site of the immunosuppressive receptor LILRB4.

20. An anti-fibronectin antibody or a derivative thereof, which binds to the amino acid sequence represented by SEQ ID NO: 1.

21. A fibronectin analog, in which any one of the following peptides (a) to (c) and an Fc region of immunoglobulin G are fused with each other:

(a) a peptide containing the amino acid sequence represented by SEQ ID NO: 1;
(b) a peptide containing an amino acid sequence in which one to several amino acids have been deleted, inserted, substituted, or added in the amino acid sequence represented by SEQ ID NO: 1, and having a binding capacity to a fibronectin binding site of the immunosuppressive receptor LILRB4; and
(c) a peptide containing an amino acid sequence having 80% or more identity to the amino acid sequence represented by SEQ ID NO: 1, and having a binding capacity to a fibronectin binding site of the immunosuppressive receptor LILRB4.

22. A kit for detecting fibronectin containing the amino acid sequence represented by SEQ ID NO: 1 or a partial protein thereof contained in a biological sample, comprising the anti-fibronectin antibody or a derivative thereof according to Claim 20.

23. A method for detecting fibronectin or a partial protein thereof in a biological sample using the kit according to Claim 22.

[FIG. 1]

EP 4 014 994 A1

[FIG. 2]

[FIG. 3]

[FIG. 4]

[FIG. 5]

[FIG. 6]

[FIG. 7]

[FIG. 8]

[FIG. 9]

## MOUSE CANCER CELL LINE

B16F10
(Melanoma)

3LL
(Lewis Lung
Carcinoma)

Isotype control
— anti-FN30 mAb No.5

## HUMAN CANCER CELL LINE

Daudi
(Burkitt's
Lymphoma)

HL60
(Promyelocytic
Leukemia)

HeLa
(Cervix
Epithelioid
Carcinoma)

HepG2
(Hepatocellular
Carcinoma)

Saos-2
(Osteosarcoma)

EP 4 014 994 A1

[FIG. 10]

[FIG. 11]

[FIG. 12]

ANTI-FN POLYCLONAL ANTIBODY

ANTI-FN30 ANTIBODY
(ANTIBODY NO. 4)

ANTI-FN30 ANTIBODY
(ANTIBODY NO. 5)

[FIG. 13]

ANTI-FN30
ANTIBODY

CALIBRATION
CURVE

$y=537.8014+(-0.09311525-537.8014)/(1+(x/801216300)^{0.3740341})$

|  | SPECIMEN 1 | SPECIMEN 2 | SPECIMEN 3 | SPECIMEN 4 |
|---|---|---|---|---|
| A | 270.44 | 401.28 | 228.8 | 237.2 |
|  | 300.52 | 374.08 | 219.78 | 259.06 |
| Average | 285.48 | 387.68 | 224.29 | 248.13 |

ANTI-FN44
ANTIBODY

CALIBRATION
CURVE

$y=873.7667+(0.3501713-873.7667)/(1+(x/331162100)^{0.4707933})$

|  | SPECIMEN 1 | SPECIMEN 2 | SPECIMEN 3 | SPECIMEN 4 |
|---|---|---|---|---|
| B | 351.72 | 448.8 | 260.46 | 321.48 |
|  | 270.8 | 371.24 | 377.88 | 327.6 |
| Average | 311.26 | 410.02 | 319.17 | 324.54 |

[FIG. 14]

**FN conc.** / **24kDa conc.**

Median: 260.76μg/ml
Average: 279.91μg/ml
Standard Deviation: 65.56μg/ml
Coefficient of variation: 0.57

Median: 6.04μg/ml
Average: 6.49μg/ml
Standard Deviation: 3.72μg/ml
Coefficient of variation: 0.23

(Boxplot representing first, second and third quartiles)

[FIG. 15]

CONTROL IgG / FN30-Fc

[FIG. 16]

[FIG. 17A]

[FIG. 17B]

EP 4 014 994 A1

[FIG. 17C]

[FIG. 17D]

[FIG. 18A]

39

[FIG. 18B]

[FIG. 19]

[FIG. 20A]

[FIG. 20B]

[FIG. 20C]

[FIG. 20D]

[FIG. 20E]

[FIG. 20F]

[FIG. 20G]

[FIG. 21]

[FIG. 22]

[FIG. 23]

control    OTs/total= 7.6%    α-LILRB4 Ab    OTs/total= 13.7%

[FIG. 24]

[FIG. 25]

WT
♀ birth 9/12~14(18weeks)

gp49B -/-
♀ birth 9/12(18weeks)

| | | |
|---|---|---|
| **INTERNATIONAL SEARCH REPORT** | | International application No. |
| | | PCT/JP2020/030175 |

**A. CLASSIFICATION OF SUBJECT MATTER**
A61K 39/395(2006.01)i; A61K 45/00(2006.01)i; A61P 19/08(2006.01)i; A61P 29/00(2006.01)i; A61P 35/00(2006.01)i; A61P 35/04(2006.01)i; A61P 37/06(2006.01)i; A61P 37/08(2006.01)i; A61P 43/00(2006.01)i; C07K 14/78(2006.01)i; C07K 16/00(2006.01)i; C07K 16/28(2006.01)i; C07K 16/30(2006.01)i; C07K 19/00(2006.01)i; C12M 1/34(2006.01)i; C12N 15/12(2006.01)i; C12Q 1/04(2006.01)i; A61K 38/10(2006.01)i; G01N 33/53(2006.01)i
FI:      A61K45/00; C07K14/78; C07K16/28; C07K16/30; A61P35/00; A61P37/06;
         A61P37/08; A61P29/00; A61P35/04; A61P19/08; A61P43/00 111;
         A61K39/395 D; A61K39/395 N; A61K38/10; G01N33/53 D; C12M1/34 F;
         C07K19/00; C07K16/00; C12Q1/04; C12N15/12 ZNA
According to International Patent Classification (IPC) or to both national classification and IPC

**B. FIELDS SEARCHED**
Minimum documentation searched (classification system followed by classification symbols)
A61K39/395;  A61K45/00;  A61P19/08;  A61P29/00;  A61P35/00;  A61P35/04;
A61P37/06;  A61P37/08;  A61P43/00;  C07K14/78;  C07K16/00;  C07K16/28;
C07K16/30; C07K19/00; C12M1/34; C12N15/12; C12Q1/04; A61K38/10; G01N33/53

Documentation searched other than minimum documentation to the extent that such documents are included in the fields searched
| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)
WPI; JSTPlus/ MEDPlus/JST7580 (JDreamIII);
CAplus/REGISTRY/MEDLINE/EMBASE/BIOSIS/WPIDS (STN)

**C. DOCUMENTS CONSIDERED TO BE RELEVANT**

| Category* | Citation of document, with indication, where appropriate, of the relevant passages | Relevant to claim No. |
|---|---|---|
| X | JP 2008-514621 A (BIOXELL S.P.A.) 08.05.2008 (2008-05-08) claims 51, 52, paragraphs [0013], [0130] | 13-16 |
| X | MORI, Y. et al., "Inhibitory Immunoglobulin-Like Receptors LILRB and PIR-B Negatively Regulate Osteoclast Development.", J. Immunol., 2008, vol. 181, no. 7, pp. 4742-4751 abstract, page 4750, right column, paragraph [0001] | 13-16 |

☒ Further documents are listed in the continuation of Box C.   ☒ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 11 September 2020 (11.09.2020) | 24 September 2020 (24.09.2020) |

| Name and mailing address of the ISA/ | Authorized officer |
|---|---|
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

C (Continuation). DOCUMENTS CONSIDERED TO BE RELEVANT

| Category* | Citation of document, with indication, where appropriate, of the relevant passages | Relevant to claim No. |
|---|---|---|
| X | US 2015/0174203 A1 (ICAHN SCHOOL OF MEDICINE AT MOUNT SINAI) 25.06.2015 (2015-06-25) claim 20 | 17-19 |
| X | JP 63-219393 A (TAKARA SHUZO CO.) 13.09.1988 (1988-09-13) claim 1, page 4, upper left column, last paragraph, upper right column, table 1, last paragraph | 20 |
| Y | | 20-23 |
| Y | SEKIGUCHI, K. et al., "Differences in domain structure between pericellular matrix and plasma fibronectins as revealed by domain-specific antibodies combined with limited proteolysis and S-cyanylation: A preliminary note.", Biochemical and Biophysical Research Communications, 1983, vol. 116, no. 2, pp. 534-540 page 537, paragraph [0002] | 20-23 |
| A | WO 2018/234367 A1 (INSTITUT CURIE) 27.12.2018 (2018-12-27) claims 1, 5, 6 | 1-23 |
| P, A | WO 2019/185792 A1 (PHILOGEN S.P.A) 03.10.2019 (2019-10-03) | 1-23 |
| A | XU, Z. et al., "ILT3.Fc-CD166 Interaction Induces Inactivation of p70 S6 Kinase and Inhibits Tumor Cell Growth.", Journal of Immunology, 2018, vol. 200, no. 3, pp. 1207-1219 entire text | 1-23 |
| A | 森優他，LILRB4 によるヒト破骨細胞の分化調節機構の解析，日本整形外科学会雑誌，2007, vol. 81, no. 8, pp. S980, 1-Pf-2 entire text, non-official translation (MORI, Yu et al., "Analysis of differentiation regulation mechanism of human osteoclast cells by LILRB4", The Journal of Japanese Orthopaedic Surgical Society) | 1-23 |

Form PCT/ISA/210 (continuation of second sheet) (January 2015)

**INTERNATIONAL SEARCH REPORT**

International application No.

PCT/JP2020/030175

| Box No. II | Observations where certain claims were found unsearchable (Continuation of item 2 of first sheet) |

This international search report has not been established in respect of certain claims under Article 17(2)(a) for the following reasons:

1. ☐ Claims Nos.:
   because they relate to subject matter not required to be searched by this Authority, namely:

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

| Box No. III | Observations where unity of invention is lacking (Continuation of item 3 of first sheet) |

This International Searching Authority found multiple inventions in this international application, as follows:
   See extra sheet

1. ☐ As all required additional search fees were timely paid by the applicant, this international search report covers all searchable claims.

2. ☒ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest**
   ☐ The additional search fees were accompanied by the applicant's protest and, where applicable, the payment of a protest fee.
   ☐ The additional search fees were accompanied by the applicant's protest but the applicable protest fee was not paid within the time limit specified in the invitation.
   ☐ No protest accompanied the payment of additional search fees.

Form PCT/ISA/210 (continuation of first sheet (2)) (January 2015)

```
<Continuation of Box No. III>
```

Document A: WO 2018/234367 A1 (INSTITUT CURIE) 27.12.2018 (2018-12-27) claims 1, 5, 6 (Family: none)
Document 4: JP 63-219393 A (TAKARA SHUZO CO.) 13.09.1988 (1988-09-13) claim 1, page 4, upper left column, last paragraph, upper right column, table 1, last paragraph & DE 3743402 A1 p. 4, lines 1-3, 20-22, table 1
Document 5: SEKIGUCHI, K. et al., "Differences in domain structure between pericellular matrix and plasma fibronectins as revealed by domain-specific antibodies combined with limited proteolysis and S-cyanylation: A preliminary note.", Biochemical and Biophysical Research Communications, 1983, vol. 116, no. 2, pp. 534-540 page 537, paragraph [0002]

Document A discloses an inhibitor of H3K9 histone methyl transferase SUV39H1 for use in combination with at least one modulator of an immune checkpoint molecule/protein in the treatment of cancer (claim 1 of claims), wherein: said at least one immune checkpoint molecule is an inhibitory immune checkpoint molecule and/or a stimulatory immune checkpoint (claim 5 of claims); and the inhibitory immune checkpoint protein is selected from PD-L1, PD1, ILT3, etc. (claim 6 of claims), and thus ILT3 corresponds to LILRB4.
Document 4 discloses an anti-human fibronectin monoclonal antibody that is characterized by specifically reacting with essentially all the human fibronectins produced from a hybridoma produced by fusion of spleen cells and myeloma cells of a mammal immunized with human fibronectin (claim 1 of claims), wherein monoclonal antibodies, which specifically bind to domain 6 from a hybridoma FN4, to domain 1 from a hybridoma FN9, and to domain 4 from hybridomas FNI0 and FN30, are obtained (page 4, upper right column, table 1, last paragraph).
Document 5 cited in document 4 (page 4, upper left column, last paragraph) indicates that N-terminal Hep-1/Fib-1 domain of fibronectin is 24K fragment (page 537, paragraph [0002]).
Since from the disclosure of document 5, domain 1 having a molecular weight of 24K which binds to heparin and fibrin set forth in document 4 is recognized to be an N-terminal domain, and the monoclonal antibody binding to domain 1 obtained from a hybridoma FN9 set forth in document 4 is recognized to bind to an N-terminal 24K domain, the antibody set forth in document 4 is highly likely to bind to an amino acid sequence part of SEQ ID NO:1 present in the N-terminal side of fibronectin.

Form PCT/ISA/210 (extra sheet) (January 2015)

**INTERNATIONAL SEARCH REPORT**

International application No.

PCT/JP2020/030175

(Invention 1) Claims 1-12
In claim 1, a novel technical feature is an immune checkpoint inhibitor using a material which inhibits the binding of fibronectin and LILRB4. Therefore, claims 1 and 12 having said technical feature are classified as invention 1.

(Invention 2) Claims 13-19
The invention in claim 13 has a material that activates LILRB4 as a therapeutic agent of immune checkpoint-associated diseases, and the invention in claim 1 and the invention in claim 13 share the common technical feature of having LILRB4 as a target of the therapeutic agent of immune checkpoint-associated diseases, but said technical feature does not make a contribution over the prior art in light of the disclosure of document A, and thus cannot be considered a special technical feature. Also, there are no other identical or corresponding special technical features between these inventions.
Furthermore, claim 13 is not dependent on claim 1. In addition, claim 13 is not substantially identical or equivalent to any of the claims classified as invention 1.
Therefore, claim 13 cannot be classified as invention 1, and thus the invention in claims 13-19 is classified as invention 2.

(Invention 3) Claims 20-23
The invention as in claim 1 and the invention as in claim 20 share the common technical feature of an "anti-fibronectin antibody." However, said technical feature does not make a contribution over the prior art in light of the disclosure of document 4, and thus cannot be considered a special technical feature. Also, there are no other identical or corresponding special technical features between these inventions.
Furthermore, claim 20 is not dependent on claim 1. In addition, claim 20 is not substantially identical or equivalent to any of the claims classified as invention 1.
Therefore, claim 20 cannot be classified as invention 1, and thus the invention in claims 20-23 is classified as invention 3.

Form PCT/ISA/210 (extra sheet) (January 2015)

**INTERNATIONAL SEARCH REPORT**
Information on patent family members

| | | International application No. |
|---|---|---|
| | | PCT/JP2020/030175 |

| Patent Documents referred in the Report | Publication Date | Patent Family | Publication Date |
|---|---|---|---|
| JP 2008-514621 A | 08 May 2008 | WO 2006/036813 A2 claims 51, 52, page 4, lines 12-20, page 37, lines 3-8 EP 1797033 A2 CA 2580962 A1 CN 101106985 A ZA 200703310 A AU 2005289664 A US 2008/0318911 A1 | |
| US 2015/0174203 A1 | 25 Jun. 2015 | WO 2013/181438 A2 | |
| JP 63-219393 A | 13 Sep. 1988 | DE 3743402 A1 page 4, lines 1-3, 20-22, table 1 | |
| WO 2018/234367 A1 | 27 Dec. 2018 | (Family: none) | |
| WO 2019/185792 A1 | 03 Oct. 2019 | GB 201805138 D GB 201812127 D | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2019148423 A
- JP 2018025554 A

**Non-patent literature cited in the description**

- *J Immunol.,* 01 February 2018, vol. 200 (3), 1207-1219
- *Blood,* 07 August 2014, vol. 124 (6), 924-935
- *Nature,* October 2018, vol. 562 (7728), 605-609
- *Rheumatology (Oxford),* July 2007, vol. 46 (7), 1071-1075
- *J Rheumatol,* October 1987, vol. 14 (5), 1052-1054
- *Rheumatol Int,* January 2013, vol. 33 (1), 37-43
- *J Cancer,* 21 July 2009, vol. 101 (2), 327-334
- **YOSHIAKI NAGAMUNE ; HIROSHI TERADA.** Monoclonal Antibody. Hirokawa Shoten Co, 1990
- **JAME W. GOLDING.** Monoclonal Antibody. Academic Press, 1996
- *Nature,* 12 March 1992, vol. 356, 152-154
- *J. Immunol Methods Mar,* vol. 1 (249), 147-154
- Technique for Preparation of Thin Frozen Sections from Undecalcified Hard Tissues. Kawamoto's method. 2008
- **TADAFUMI KAWAMOTO.** *Pathological Technology,* 2009, vol. 72 (2), 76-83